# NEUE EUROPÄISCHE PATENTSCHRIFT

(11) **EP 0 861 236 B2**
(45) Veröffentlichungstag und Bekanntmachung des Hinweises auf die Entscheidung über den Einspruch: **16.08.2006**
(45) Hinweis auf die Patenterteilung: 13.02.2002
(21) Anmeldenummer: 96938052.6
(22) Anmeldetag: 04.11.1996
(51) Int. Cl.: C07D 217/26, A61K 31/47, C07D 405/12

(54) **CYCLISCHE UND HETEROCYCLISCHE N-SUBSTITUIERTE ALPHA-IMINOHYDROXAM- UND CARBONSÄUREN**
CYCLIC AND HETEROCYCLIC N-SUBSTITUTED ALPHA-IMINOHYDROXAMIC AND CARBOXYLIC ACIDS
ACIDES ALPHA-IMINOHYDROXAMIQUES ET CARBOXILIQUES CYCLIQUES ET HETEROCYCLIQUES SUBSTITUES EN POSITION N

(30) Priorität: 13.11.1995 DE 19542189; 28.03.1996 DE 19612298
(43) Veröffentlichungstag der Anmeldung: 02.09.1998
(73) Patentinhaber: Sanofi-Aventis Deutschland GmbH, 65929 Frankfurt am Main (DE)
(72) Erfinder: THORWART, Werner, D-65239 Hochheim (DE); SCHWAB, Wilfried, D-65207 Wiesbaden (DE); SCHUDOK, Manfred, D-65817 Eppstein (DE); HAASE, Burkhard, D-63477 Maintal (DE); BARTNIK, Eckart, D-65205 Wiesbaden (DE); WEITHMANN, Klaus-Ulrich, D-65719 Hofheim (DE)
(86) Internationale Anmeldenummer: PCT/EP1996/004776
(87) Internationale Veröffentlichungsnummer: WO 1997/018194

(56) Entgegenhaltungen:
- EP-A- 0 468 231
- EP-A- 0 606 046
- EP-A- 0 614 911
- WO-A-95/35276
- WO-A-96/00214
- DE-A- 2 125 778
- JP-A- 4 210 675
- US-A- 5 219 851
- CHEMICAL ABSTRACTS, vol. 112, no. 9, 26.Februar 1990 Columbus, Ohio, US; abstract no. 76983z, XP002023924 & INDIAN J.CHEM.,SECT.B, Bd. 28B, Nr. 4, 1989, Seiten 333-337, RAVISH C. TRIPATHI ET AL: "Synthesis and SAR studies in 2-substituted 1,2,3,4-tetrahydro-9H-pyrido[3,4-b]indole- 3-carboxylic acids-a new class of potent antiulcer agents"
- European Polymer Journal (1972), 8, pp. 513-524
- Aust. J. Chem. (1967), 20 (9), pp. 1935-1941
- Biorg. Med. Chem. Lett (1994), 4(16), pp. 2029-2572.
- J. Med. Chem. (1992), 35(14), pp. 2562-2572.
- Yakugaku Zasshi (1974), 94(8), pp. 934-944.

## Beschreibung

Die Erfindung betrifft cyclische und heterocyclische N-substituierte α-Iminohydroxam- und carbonsäuren, Verfahren zu ihrer Herstellung und Verwendung derselben als Arzneimittel.

In EP 0 606 046 werden einige Arylsulfonamido-hydroxamsäurederivate beschrieben und deren Wirkung als Matrix-Metalloproteinase-Inhibitoren.

In dem Bestreben weiterewirksame Verbindungen zur Behandlung von Bindegewebserkrankungen zu finden, wurde nun gefunden, daß die erfindungsgemäßen Iminohydroxamsäurederivate Inhibitoren von Metalloproteinasen sind.

Die Erfindung betrifft eine Verbindung der Formel I 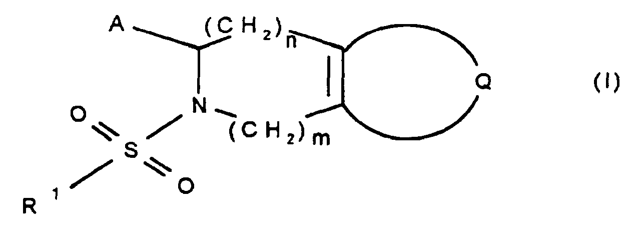 und/oder eine gegebenenfalls stereoisomere Form der Verbindung der Formel I und/oder ein physiologisch verträgliches Salz der Verbindung der Formel I, wobei für den Fall i)
R¹ für
a) einen Rest der Formel II 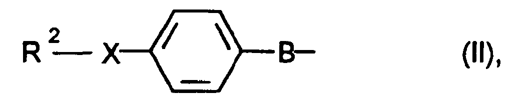
b) einen Rest der Formel III 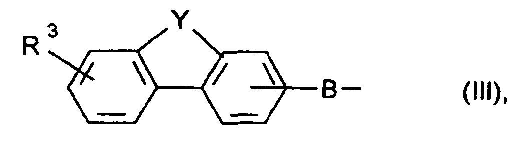
c) einen Rest der Formel IV 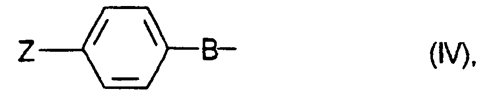 worin Z ein Rest eines Heterocyclus oder substituierten Heterocyclus ist, wie
   1) Pyrrol,
   2) Thiazol,
   3) Pyrazol,
   4) Pyridin
   5) Imidazol,
   6) Pyrrolidin,
   7) Piperidin,
   8) Thiophen,
   9) Oxazol,
   10) Isoxazol,
   11) Morpholin oder
   12) Piperazin,
   steht, oder
d) einen Rest der Formel V 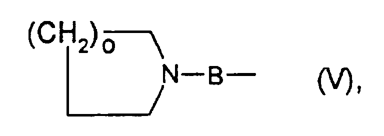 worin o die Zahl 1 oder 2 ist, und eines der Kohlenstoffatome im Ring gegebenenfalls durch -O- oder -S- ersetzt ist, bedeutet, und
Q als Teil der Strukturformel I 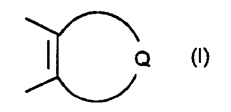
1) für den Strukturteil VI 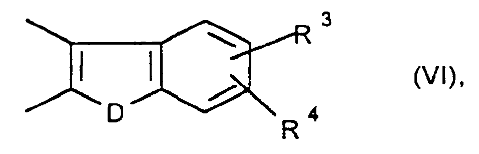
2) den Strukturteil VII 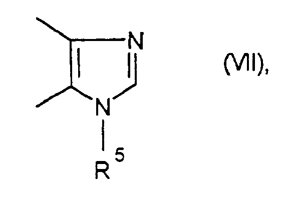
3) für den Strukturteil VIII 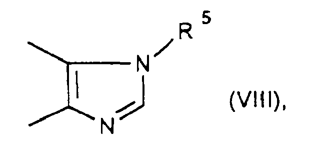
4) den Strukturteil IX 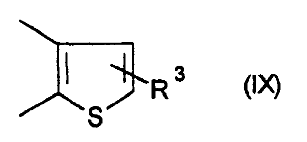 oder
5) für den Strukturteil X 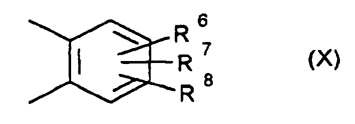 steht, worin D NR⁴ oder S bedeutet,
   R² für

1) Phenyl oder
2) Phenyl ein- bis dreifach substituiert durch
   2.1 Hydroxy,
   2.2 -O-R¹⁰ worin R¹⁰
      1) (C₁-C₆)-Alkyl,
      2) (C₃-C₆)-Cycloalkyl,
      3) Benzyl oder
      4) Phenyl bedeutet,
   2.3 -COOH,
   2.4 (C₁-C₆)-Alkyl,
   2.5 (C₃-C₆)-Cycloalkyl-O-(C₁-C₄)-alkyl,
   2.6 Halogen,
   2.7 -CN,
   2.8 -NO₂,
   2.9 -CF₃,
   2.10 -O-C(O)-R¹⁰ und R¹⁰ wie oben definiert ist,
   2.11 -O-C(O)-Phenyl, ein- oder zweifach substituiert durch R³,
   2.12 -C(O)-O-R¹⁰ und R¹⁰ wie oben definiert ist,
   2.13 Methylendioxo,
   2.14 -C(O)-NR¹¹R¹², worin
      R¹¹ und R¹² gleich oder verschieden sein können und
      1) Wasserstoffatom,
      2) (C₁-C₄)-Alkyl oder
      3) Benzyl bedeuten oder
      4) R¹¹ und R¹² zusammen mit dem N-Atom an das sie gebunden sind einen Pyrrolidin-, Piperidin-, Morpholin- oder Piperazinrest bilden, oder
   2.15. -NR¹³R¹⁴, worin
      R¹³ Wasserstoffatom oder (C₁-C₄)-Alkyl bedeutet und
      R
      ¹⁴ 1) Wasserstoffatom,
      2) (C₁-C₄)-Alkyl,
      3) Benzyl,
      4) -C(O)-R¹⁰ oder
      5) -C(O)-O-R¹⁰ bedeutet und R¹⁰ wie oben definiert ist,
R³ und R⁴ gleich oder verschieden sind und
1) Wasserstoffatom,
2) (C₁-C₅)-Alkyl,
3) (C₁-C₅)-Alkoxy,
4) Halogen,
5) Hydroxy,
6) -O-C(O)-R¹⁰ und R¹⁰ wie oben definiert ist, bedeuten, oder
7) R³ und R⁴ zusammen den Rest-O-CH₂-O- bilden,
R⁵ für
a) Wasserstoffatom,
b) (C₁-C₅)-Alkyl oder
c) Benzyl steht, und
R⁶, R⁷ und R⁸ gleich oder verschieden sind und für
a) Wasserstoffatom stehen, oder
b) die Bedeutung für den Fall i) von R² unter den Punkten 2.1 bis 2.14, haben, und
n für Null,1 oder 2 steht,
m für Null,1 oder 2 steht, wobei die Summe von n und m 1, 2 oder 3 ist,
mit Ausnahme des Falles, dass
A für HO-C(O)- steht, Q als Teil der Strukturformel I für den Strukturteil X steht, R⁶, R⁷ und R⁸ für Wasserstoffatom oder -O-Methyl stehen, n für 1 steht, m für 1 steht und R¹ für den Rest der Formel V steht, worin o die Zahl 2 ist und eines der Kohlenstoffatome durch -O- ersetzt ist, oder
wobei für den Fall ii)
R¹ für
1) Phenyl oder
2) Phenyl, ein- bis dreifach substituiert durch R², wobei R² wie für den Fall i) unter den Punkten 2.1 bis 2.15 definiert ist,
Q für den Strukturteil X steht und
R⁶, R⁷ und R⁸ gleich oder verschieden sind und wie oben definiert sind,
n 1 bedeutet und
m 1 bedeutet,
mit Ausnahme der Fälle, dass
A für HO-C(O)- steht, R⁶, R⁷ und R⁸ für Wasserstoffatom stehen, n für 1 steht, m für 1 steht und R¹ für Phenyl steht, das einfach durch Methyl, Nitro oder Amino substituiert ist oder
A für HO-C(O)- steht, R⁶, R⁷ und R⁸ für Wasserstoffatom stehen, n für 1 steht, m für 1 steht und R¹ für Phenyl steht, das zweifach durch Amino und Chlor substituiert ist oder
wobei für den Fall iii)
R¹, Q, R⁶, R⁷ und R⁸ gleich oder verschieden sind und die für den Fall ii) genannte Bedeutung haben, m und n Null, 1 oder 2 bedeuten und wobei die Bedeutung von n und m nicht gleich ist,
mit Ausnahme des Falles, dass
A für HO-C(O)- steht, R⁶, R⁷ und R⁸ für Wasserstoffatom stehen, n für 1 steht, m für Null steht und R¹ für Phenyl steht, das einfach durch Methyl substituiert ist und
X für
a) eine kovalente Bindung,
b) -O-,
c) -S-,
d) -S(O)-,
e) -S(O)₂-,
f) -C(O)- oder
g) -C(OH)- steht, und
Y für
a) -O- oder
b) -S- steht, und
A für HO-NH-C(O)- oder HO-C(O)- steht und
B für a) -(CH₂)_{q}-, worin q Null,1, 2, 3 oder 4 bedeutet, oder b) -CH=CH- steht.

Bevorzugt ist eine Verbindung der Formel I und/oder ein physiologisch verträgliches Salz der Verbindung der Formel I und/oder eine gegebenenfalls stereosiomere Form der Verbindung der Formel I, wobei
R¹ für den Fall i) für einen Rest der Formel II oder III steht und Q für den Strukturteil VI, VII, VIII oder X steht,
R¹ für den Fall ii) für Phenyl oder Phenyl, ein- bis dreifach substituiert durch Methoxy, steht und Q für den Strukturteil X steht, oder
R¹ für den Fall iii) für Phenyl, Q für den Strukturteil X steht, n Null und m 2 bedeutet, und
A für HO-NH-C(O)- oder HO-C(O)- steht,
B für eine kovalente Bindung steht,
X für Sauerstoffatom oder eine kovalente Bindung steht, und
R² für Phenyl oder Phenyl substituiert durch
a) Hydroxy,
b) -O-R¹⁰, worin R¹⁰ (C₁-C₃)-Alkyl oder Benzyl bedeutet,
c) (C₁-C₂)-Alkyl,
d) Fluor oder Chlor,
e) -CN,
f) -CF₃ oder
g) -NR¹³R¹⁴, worin R¹³ und R¹⁴ (C₁-C₃)-Alkyl bedeuten, steht
R³, R⁴, R⁵, R⁶, R⁷ und R⁸ gleich oder verschieden sind und für
a) Wasserstoffatom,
b) Methoxy,
c) Methylendioxo,
d) Amino oder
e) Hydroxy stehen.

Insbesondere bevorzugt sind die Verbindungen
R-2-(Biphenylsulfonyl)-1,2,3,4-tetrahydroisochinolin-3-hydroxamsäure,
R-2-(4-Chlor-biphenylsulfonyl)-1,2,3,4-tetrahydroisochinolin-3-hydroxamsäure,
R-2-(4-Chlor-biphenylsulfonyl)-1,2,3,4-tetrahydroisochinolin-3-carbonsäure,
R-2-(4-Phenoxybenzolsulfonyl)-1,2,3,4-tetrahydroisochinolin-3-hydroxymsäure,
R-2-(4-Phenoxybenzolsulfonyl)-1,2,3,4-tetrahydroisochinolin-3-carbonsäure,
R-2-(4-(4-Dimethylaminophenoxy)-benzolsulfonyl)-1,2,3,4-tetrahydroisochinolin-3-hydroxamsäure,
R-2-(4-Dimethylaminobiphenylsulfonyl)-1,2,3,4-tetrahydroisochinolin-3-carbonsäure,
R-2-(4-Benzoylphenylsulfonyl)-1,2,3,4-tetrahydroisochinolin-3-hydroxamsäure,
R-2-(4-Methoxybenzolsulfonyl)-7-hydroxy-1,2,3,4-tetrahydroisochinolin-3-hydroxamsäure,
R-2-(4-Methoxybenzolsulfonyl)-7-nitro-1,2,3,4-tetrahydroisochinolin-3-hydroxamsäure,
2-(4-Methoxybenzolsulfonyl)-6,7-propylen-1,2,3,4-tetrahydroisochinolin-1-hydroxamsäure,
R-5-(4-Methoxybenzolsulfonyl)-4,5,6,7-tetrahydro-1 H-imidazo-(4,5-c)-pyridin-6-hyd roxamsäure,
R-2-(4-Methoxybenzolsulfonyl)-1,2,3,4-tetrahydro-9H-pyrido-(3,4-c)-indol-3-hydroxamsäure,
R-2-(4-Phenoxybenzolsulfonyl)-1,2,3,4-tetrahydro-9H-pyrido-(3,4-c)-indol-3-hydroxamsäure.

Weiterhin sind diejenigen Verbindungen der Formel I besonders hervorzuheben, in denen das zentrale Kohlenstoffatom zwischen Amino- und Säuregruppe als R-Enantiomer vorliegt.

Unter dem Begriff Halogen wird Fluor, Chlor, Brom oder Jod verstanden. Unter dem Begriff Alkyl oder Alkoxy werden Reste verstanden deren Kohlenstoffkette geradkettig, verzweigt oder cyclisch sein kann. Cyclische Alkylreste sind beispielsweise 3- bis 6-gliedrige Monocyclen wie Cyclopropyl, Cyclobutyl, Cyclopentyl oder Cyclohexyl.

Zu den "Heterocyclen der Formel V" gehören beispielsweise Thiomorpholin, Piperidin, Morphoiin oder Piperazin.

Geeignete physiologisch verträgliche Salze der Verbindung der Formel sind beispielsweise Alkali-, Erdalkali-und Ammoniumsalze einschließlich solcher von organischen Ammoniumbasen oder basische Aminosäuren.

Die Erfindung betrifft auch ein Verfahren zur Herstellung der Verbindung der Formel I und/oder ein physiologisch verträgliches Salz der Verbindung der Formel I und/oder eine gegebenenfalls stereoisomere Form der Verbindung der Formel I, das dadurch gekennzeichnet ist, daß man
a) eine Iminosäure der Formel XI 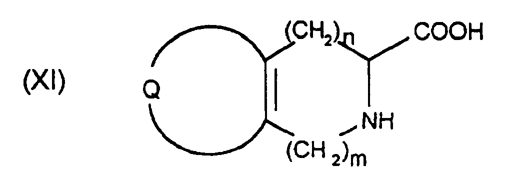 worin Reste Q, sowie n und m wie in Formel I definiert sind, mit einem (C₁-C₄)-Alkohol oder einem Benzylalkohol zu der Verbindung der Formel XII umsetzt, 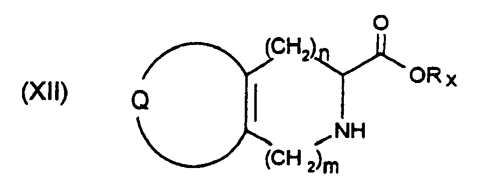 worin Rₓ (C₁-C₄)-Alkyl oder Benzyl bedeutet, oder
b) eine nach Verfahren a) hergestellte Verbindung der Formel XII mit der Verbindung der Formel XIII, 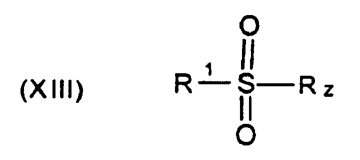 worin R¹ wie in Formel I definiert ist und R_{z} Chloratom, Imidazolyl oder-OH bedeutet, in Gegenwart einer Base oder gegebenenfalls eines wasserentziehenden Mittels zu einer Verbindung der Formel XIV umsetzt, 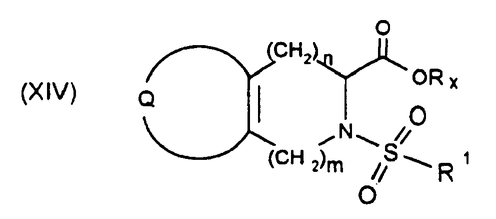 worin Q, R¹, n und m wie in Formel I definiert sind und Rₓ wie in Formel XII definiert ist, oder
c) eine nach Verfahren a) hergestellte Verbindung der Formel XII mit einer Base umsetzt und anschließend mit einer Verbindung der Formel XIII zu einer Verbindung der Formel XIV umsetzt, oder
d) eine Verbindung der Formel XI mit einer Verbindung der Formel XII zu einer Verbindung der Formel XV umsetzt 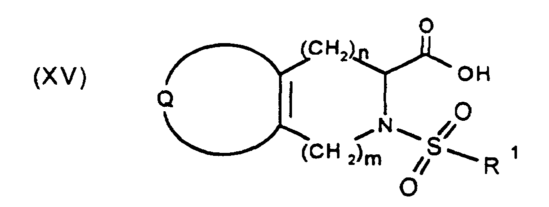 worin Q, R¹, n und m wie in Formel I definiert sind, oder
e) eine Verbindung der Formel XIV zu Verbindung der Formel XV umsetzt, oder
f) eine nach Verfahren b) oder c) hergestellte Verbindung der Formel XIV mit dem Hydroxylamin der Formel XVI,

   (XVI) H₂N-OR_{y}

   worin Ry Wasserstoffatom oder eine Sauerstoffschutzgruppe bedeutet, zu der Verbindung der Formel I umsetzt und gegebenenfalls die Sauerstoffschutzgruppe abspaltet, oder
g) eine nach Verfahren d) oder e) hergestellte Verbindung der Formel XV mit dem Hydroxylamin der Formel XVI zu der Verbindung der Formel I umsetzt, oder
h) eine nach den Verfahren f) oder g) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppe in die reinen Enantiomeren auftrennt, oder
i) die nach den Verfahren f), g) oder h) hergestellte Verbindung der Formel I entweder in freier Form isoliert oder im Falle des Vorliegens von sauren oder basischen Gruppen gegebenenfalls in physiologisch verträgliche Salze umwandelt.

Die Umsetzung gemäß Verfahrensschritt a) erfolgt für den Fall der (C₁-C₄)-Alkohole in Gegenwart von HCl-Gas oder Thionylchlorid unter üblichen Reaktionsbedingungen. Die Herstellung der entsprechenden Benzylester der Formel XII erfolgt in Benzol oder Toluol mit dem entsprechenden Alkohol sowie einer Säure wie p-Toluolsulfonsäure. Tertiärbutylester lassen sich beispielsweise nach bekannten Verfahren mit Isobuten und Schwefelsäure herstellen.

Die Umsetzung gemäß Verfahrensschritt b) erfolgt in Gegenwart einer basischen Verbindung wie N-Methylmorpholin (NMM), N-Ethylmorpholin (NEM), Triethylamin (TEA), Diisopropylethylamin (DIPEA), Pyridin, Collidin, Imidazol oder Natriumcarbonat in Lösungsmitteln wie Tetrahydrofuran (THF), Dimethylformamid (DMF), Dimethylacetamid, Dioxan, Acetonitril, Toluol, Chloroform oder Methylenchlorid, oder auch in Gegenwart von Wasser. Bevorzugt werden die Sulfonsäurechloride der Formel XIII in Gegenwart von NMM in THF eingesetzt.

Die Umsetzung gemäß Verfahrensschritt c) erfolgt in Gegenwart einer Base wie KOH, LiOH oder NaOH.

Die Umsetzung gemäß Verfahrensschritt d) erfolgt in einem wäßrig organischen Lösungsmittelsystem, bevorzugt in THF und Wasser in Gegenwart von einer Base wie Natriumcarbonat und der Verbindung der Formel XIII. Ferner kann die Umsetzung ohne Lösungsmittel mit oder ohne Base unter vermindertem Druck, wie er durch eine Ölpumpe erreicht wird, durchgeführt werden.

Die Verseifung der Verbindung der Formel XIV zu der Verbindung der Formel XV (Verfahrensschritt e) erfolgt beispielsweise basisch, bevorzugt sauer oder im Falle der Benzylderivate durch Hydrogenolyse. Im Fall der basischen Verseifung ist es nötig, die Carbonsäure durch Behandlung mit einer anderen Säure, z.B. verdünnter Salzsäure, aus dem Carbonsäuresalz freizusetzen.

Die Umsetzung gemäß Verfahrensschritt f) erfolgt unter den für die Bildung von Carbonsäureamiden üblichen Bedingungen in einem geeigneten Lösungsmittel, z.B. Alkohole oder Dimethylformamid.

Bei der Umsetzung gemäß Verfahrensschritt g) werden die Carbonsäuren der Formel XV aktiviert. Aktivierte Carbonsäuren sind beispielsweise Acylhalogenide, Acylazide, gemischte Anhydride und Carbonate. Bevorzugt sind Acylchloride oder -fluoride, gemischte Anhydride und Carbonate aus Pivaloylchlorid, Ethyl-, Isopropyl- oder Isobutylchloroformat; Aktivesterwie Cyanoethyl, o- oderp-Nitrophenyl, Succinimido oder Phtalimido, sowie die durch die Kupplungsreagentien wie Diisopropylcarbodiimid (DIC), Carbonyldiimidazol (CDI), Dicyclohexylcarbodiimid (DCC) oder Benzotriazolyltetramethyluronium Tetrafluoroborat (TBTU), gegebenenfalls unter Zugabe von Hydroxybenzotriazol (HObt) oder Oxohydroxybenzotriazin (HOObt), erhältlichen aktivierten Carbonsäuren, wobei als Lösungsmittel aprotische Lösungsmittel bevorzugt werden.

Die eingesetzten Ausgangsprodukte und Reagenzien können entweder nach bekannten Verfahren hergestellt werden oder sind käuflich erhältlich.

Als geeignete Iminosäuren der Formel XI, bei denen n und m eine 1 bedeuten, seine beispielsweise 1,2,3,4-Tetrahydroisochinolin-3-carbonsäure, 1,2,3,4-Tetrahydro-9H-pyrido(3,4-b)-indol-3-carbonsäure oder ggf. 1-oder 3-substituierte 4,5,6,7-Tetrahydro-1H-imidazo-(4,5-c)-pyridin-6-carbonsäuren genannt. Ihre Herstellung erfolgt bevorzugt durch Cyclisierung der entsprechenden Aminosäuren mit Formaldehyd in Gegenwart einer Säure, wie Salz-oder Schwefelsäure, nach der Methode von Pictet-Spengler (siehe W. M. Whaley, Organic Reactions 6 (1951) 151.

Für den Fall, daß bei der Iminosäure der Formel XI n eine Null und m eine 2 bedeuten, kann beispielsweise 1,2,3,4-Tetrahydro-9H-pyrido(3,4-b)indol-1-carbonsäure und 6,7-Propylen-1,2,3,4-tetraisochinolin-1-carbonsäure als Ausgangsprodukt benutzt werden. Zur Herstellung letzterer Verbindung wird Indan nach Friedel-Crafts mit Phenylsulfonylarziridin alkyliert. Die Cyclisierung des erhaltenen 4-(2-Benzolsulfonamidoethyl)indans erfolgt mit Glyoxylsäure in HBr/Eisessig; die anschließende Abspaltung des Benzolsulfonylrestes mit lod/rotem Phosphor in HBr/Eisessig.

Als Beispiel für den Fall, daß in der Verbindung Xl n eine 1 und m eine Null bedeutet, sei die Indolin-2-carbonsäure genannt. Ihre Herstellung erfolgt beispielsweise durch katalytische Hydrierung der Indol-2-carüonsäure. Weiterhin sei die Cyclisierung von 2-Chlorphenyl-alanin oder 2-Hydroxy-3-(2-chlorphenyl)-propionsäure zu Iminosäuren der Formel Xl genannt.

Sofern Verbindungen der Formel I diastereoisomere oder enantiomere Formen zulassen und bei der gewählten Synthese als deren Gemische anfallen, gelingt die Trennung in die reinen Stereoisomeren entweder durch Chromatographie an einem gegebenenfalls chiralen Trägermaterial, oder, sofern die racemische Verbindung der Formel I oder eine Verbindung der Formel XI zur Salzbildung befähigt ist, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenylethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden, und aus den so gewonnenen diastereomeren Salzen die reinen Enantiomeren gewonnen. Auf prinzipiell gleiche Weise kann man die racemischen Verbindungen der Formel 1, die eine basische Gruppe wie eine Aminogruppe enthalten, mit optisch aktiven Säuren, wie (+)-Campher-10-sulfonsäure, D- und L- Weinsäure, D-und L-Milchsäure sowie (+) und (-)-Mandelsäure in die reinen Enantiomeren überführen. Auch kann man chirale Verbindungen, die Alkohol- oder Aminfunktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführten chiralen Molekülteil mittels geeigneter Methoden wieder abspaltet.

Saure oder basische Produkte der Verbindung der Formel I können in Form ihrer Salze oder in freier -Form vorliegen. Bevorzugt sind pharmakologisch verträgliche Salze, z. B. Alkali- oder Erdalkalimetallsalze bzw. Hydrochloride, Hydrobromide, Sulfate, Hemisulfate, alle möglichen Phosphate sowie Salze der Aminosäuren, natürlicher Basen oder Carbonsäuren.

Hydroxylamin kann in freier Form, erhältlich aus Hydroxylamin-Salzen und einer geeigneten Base in Lösung- oder in O-geschützter Form bzw. jeweils auch in Form seiner Salze eingesetzt werden. Die Herstellung des freien Hydroxylamins ist literaturbekannt und kann beispielsweise in alkoholischer Lösung erfolgen. Bevorzugt ist die Verwendung des Hydrochlorids zusammen mit Alkoholaten wie Na-Methanolat, Kaliumhydroxyd oder Kalium-t-Butanolat.

O-geschützte Hydroxylamin-Derivate enthalten bevorzugt unter milden Bedingungen abspaltbare Schutzgruppen. Bevorzugt sind hier insbesondere Schutzgruppen des Silyl-, Benzyl- und Acetal-Typs. Besonders geeignet sind dabei das O-Trimethylsilyl-, O-Tertiär-butyldimethylsilyl-, O-Benzyl, O-Tertiärbutyl sowie O-Tetrahydropyranyl-Derivat.

Ausgangsverbindungen und Zwischenprodukte, die zur Herstellung der Verbindung der Formel I verwendet werden, können, sofern funktionelle Gruppen wie Hydroxyl, Thiol, Amino oder Carboxyl, z.B. bei den Resten R¹, R², R³, R⁴, R⁵, R⁶, R⁷ und R⁸, enthalten sind, in geeignet geschützter Form eingesetzt werden.

Die Einführung von Schutzgruppen ist in all denen Fällen notwendig, in denen bei einer gewünschten chemischen Reaktion an anderen als den Reaktionszentren unerwünschte Nebenreaktionen zu erwarten sind (T.W.Greene, Protective Groups in Organic Synthesis, Wiley, New York,1991).

Die eingesetzten Schutzgruppen können vor oder nach der Umsetzung der Verbindung der Formel XII zur Verbindung der Formel I abgespalten werden.

Als Hilfsstoffe und Basen können insbesondere eingesetzt werden: HObt, HOObt, N-Hydroxysuccinimid (HOSu), TEA, NMM, NEM, DIPEA, Imidazol. Bevorzugte Lösemittel für die Reaktion sind: Dichlormethan (DCM), THF, Acetonitril, N,N-Dimethylacetamid (DMA), DMF und N-Methylpyrrolidon (NMP).

Die bevorzugten Temperaturen liegen zwischen - 78 °C und + 90 °C, je nach Siedetemperatur und Art des verwendeten Lösemittels. Besonders bevorzugt ist der Temperaturbereich von -20 bis + 30 °C.

Die Herstellung physiologisch verträglicher Salze aus zur Salzbildung befähigten Verbindungen der Formel I, einschließlich deren stereoisomeren Formen, erfolgt in an sich bekannter Weise. Die Carbonsäuren und Hydroxamsäuren bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin oder Triethanolamin oder auch basischen Aminosäuren, etwa Lysin, Ornithin oder Arginin, stabile Alkali-, Erdalkali- oder gegebenenfalls substituierte Ammoniumsalze. Sofern die Verbindungen der Formeln 1 basische Gruppen aufweist, lassen sich mit starken Säuren auch stabile Säureadditionssalze herstellen. Hierfür kommen sowohl anorganische als auch organische Säuren, wie Chlorwasserstoff-, Bromwasserstoff-, Schwefel-, Phosphor-, Methansulfon-, Benzolsulfon-, p-Toluolsulfon-, 4-Brombenzol-sulfon-, Cyclohexylamidosulfon-, Trifluormethylsulfon-, Essig-, Oxal-, Wein-, Bernstein- oder Trifluoressigsäure in Frage.

Die Erfindung betrifft auch Arzneimittel, enthaltend eine wirksame Menge von mindestens einer Verbindung der Formel I 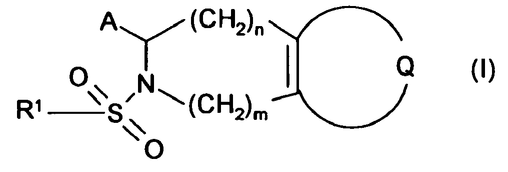 und/oder eine gegebenenfalls stereoisomere Form der Verbindung der Formel I und/oder ein physiologisch verträgliches Salz der Verbindung der Formel I, wobei für den Fall i)
R¹ für
a) einen Rest der Formel II 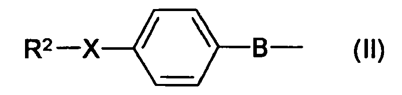
b) einen Rest der Formel III 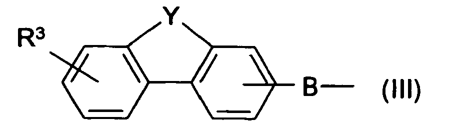
c) einen Rest der Formel IV 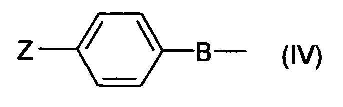 worin Z ein Rest eines Heterocyclus oder substituierten Hetereocyclus ist, wie
   1) Pyrrol,
   2) Thiazol,
   3) Pyrazol,
   4) Pyridin,
   5) Imidazol,
   6) Pyrrolidin,
   7) Piperidin,
   8) Thiophen,
   9) Oxazol,
   10) Isoxazol,
   11) Morpholin oder
   12) Piperazin, steht, oder
d) einen Rest der Formel V 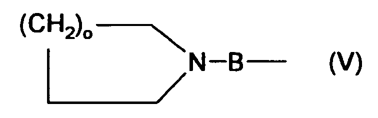 worin o die Zahl 1 oder 2 ist, und eines der Kohlenstoffatome im Ring gegebenenfalls durch -O- oder -S- ersetzt ist, bedeutet, und
Q als Teil der Strukturformel I 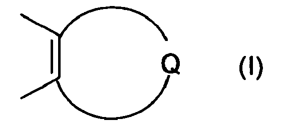
1) für den Strukturteil VI 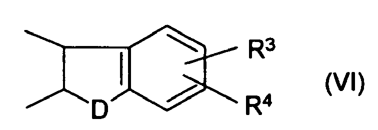
2) den Strukturteil VII 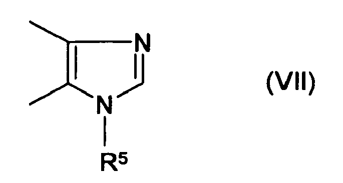
3) für den Strukturteil VIII 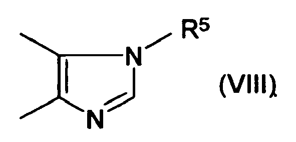
4) den Strukturteil IX 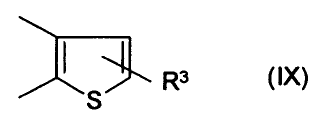 oder
5) den Strukturteil X 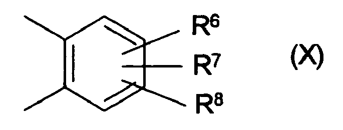 steht, worin D NR⁴ oder S bedeutet,
R² für 1) Phenyl oder
2) Phenyl ein- bis dreifach substituiert durch
   2.1. Hydroxy,
   2.2. -O-R¹⁰, worin R¹⁰
      1) (C₁-C₆)-Alkyl,
      2) (C₃-C₆)-Cycloalkyl,
      3) Benzyl oder
      4) Phenyl bedeutet,
   2.3. -COOH,
   2.4. (C₁-C₆)-Alkyl,
   2.5. (C₃-C₆)-Cycloalkyl-O-(C₁-C₄)-alkyl,
   2.6. Halogen,
   2.7. -CN,
   2.8. -NO₂,
   2.9. -CF₃,
   2.10. -O-C(O)-R¹⁰ und R¹⁰ wie oben definiert ist,
   2.11. -O-C(O)-Phenyl, ein- oder zweifach substituiert durch R³,
   2.12. -C(O)-O-R¹⁰ und R¹⁰ wie oben definiert ist,
   2.13. Methylendioxo,
   2.14. -C(O)-NR¹¹R¹², worin
      R¹¹ und R¹² gleich oder verschieden sein können und
      1) Wasserstoffatom,
      2) (C₁-C₄)-Alkyl oder
      3) Benzyl bedeuten oder
      4) R¹¹ und R¹² zusammen mit dem N-Atom an das sie gebunden sind einen Pyrrolidin-, Piperidin-, Morpholin- oder Piperazinrest bilden, oder
   2.15. -NR¹³R¹⁴, worin R¹³
      Wasserstoffatom oder (C₁-C₄)-Alkyl bedeutet und
      R¹⁴
      1) Wasserstoffatom,
      2) (C₁-C₄)-Alkyl,
      3) Benzyl,
      4) -C(O)-R¹⁰ oder
      5) -C(O)-O-R¹⁰ bedeutet und R¹⁰ wie oben definiert ist,
R³ und R⁴ gleich oder verschieden sind und
1) Wasserstoffatom,
2) (C₁-C₅)-Alkyl,
3) (C₁-C₅)-Alkoxy,
4) Halogen,
5) Hydroxy,
6) -O-C(O)-R¹⁰ und R¹⁰ wie oben definiert ist, bedeuten, oder
7) R³ und R⁴ zusammen den Rest -O-CH₂-O- bilden,
R⁵ für
a) Wasserstoffatom,
b) (C₁-C₅)-Alkyl oder
c) Benzyl steht, und
R⁶, R⁷ und R⁸ gleich oder verschieden sind und für
a) Wasserstoffatom stehen, oder
b) die Bedeutung für den Fall i) von R² unter den Punkten 2.1 bis 2.14, haben, und
n für Null, 1 oder 2 steht,
m für Null, 1 oder 2 steht, wobei die Summe von n und m 1, 2 oder 3 ist, oder
wobei für den Fall ii)
R¹ für
1) Phenyl oder
2) Phenyl, ein- bis dreifach substituiert durch R², wobei R² wie für den Fall i) unter den Punkten 2.1 bis 2.15 definiert ist,
Q für den Strukturteil X steht und
R⁶, R⁷ und R⁸ gleich oder verschieden sind und wie oben definiert sind,
n 1 bedeutet und
m 1 bedeutet, oder
wobei für den Fall iii)
R¹, Q, R⁶, R⁷ und R⁸ gleich oder verschieden sind und die für den Fall ii) genannte Bedeutung haben, m und n Null, 1 oder 2 bedeuten und wobei die Bedeutung von n und m nicht gleich ist, und
X für
a) eine kovalente Bindung,
b) -O-,
c) -S-,
d) -S(O)-,
e) -S(O)₂-,
f) -C(O)- oder
g) -C(OH)- steht, und
Y für
a) -O- oder
b) -S- steht, und
A für HO-NH-C(O)- oder HO-C(O)- steht und
B für a) -(CH₂)_{q}-, worin q Null,1, 2, 3 oder 4 bedeutet, oder b) -CH=CH- steht, neben physiologisch annehmbaren Hilfs- und Trägerstoffen, gegebenenfalls weiteren Zusatzstoffen und/oder anderen Wirkstoffen.

Die Erfindung betrifft auch Arzneimittel, enthaltend eine wirksame Menge von mindestens einer Verbindung der Formel 1, wobei
R¹ für den Fall i) für einen Rest der Formel II oder III steht und Q für den Strukturteil VI, VII, VIII oder X steht,
R¹ für den Fall ii) für Phenyl oder Phenyl, ein- bis dreifach substituiert durch Methoxy, steht und Q für den Strukturteil X steht, oder
R¹ für den Fall iii) für Phenyl, Q für den Strukturteil X steht, n Null und m 2 bedeutet, und
A für HO-NH-C(O)- oder HO-C(O)- steht,
B für eine kovalente Bindung steht,
X für Sauerstoffatom oder eine kovalente Bindung steht, und
R² für Phenyl oder Phenyl substituiert durch
a) Hydroxy,
b) -O-R¹⁰, worin R¹⁰ (C₁-C₃)-Alkyl oder Benzyl bedeutet,
c) (C₁-C₂)-Alkyl,
d) Fluor oder Chlor,
e) -CN,
f) -CF₃ oder
g) -NR¹³R¹⁴, worin R¹³ und R¹⁴ (C₁-C₃)-Alkyl bedeuten
R³, R⁴, R⁵, R⁶, R⁷ und R⁸ gleich oder verschieden sind und für
a) Wasserstoffatom,
b) Methoxy,
c) Methylendioxo,
d) Amino oder
e) Hydroxy stehen.

Die Erfindung betrifft ferner Arzneimittel, enthaltend eine wirksame Menge von mindestens einer Verbindung der Formel I, wobei man
R-2-(Biphenylsulfonyl)-1,2,3,4-tetrahydroisochinolin-3-hydroxamsäure,
R-2-(4-Chlor-biphenylsulfonyl)-1,2,3,4-tetrahydroisochinolin-3-hydroxamsäure,
R-2-(4-Chlor-biphenylsulfonyl)-1,2,3,4-tetrahydroisochinolin-3-carbonsäure,
R-2-(4-Phenoxybenzolsulfonyl)-1,2,3,4-tetrahydroisochinolin-3-hydroxymsäure,
R-2-(4-Phenoxybenzolsulfonyl)-1,2,3,4-tetrahydroisochinolin-3- carbonsäure,
R-2-(4-(4-Dimethylaminophenoxy)-benzolsulfonyl)-1,2,3,4-tetrahydroisochinolin-3-hydroxamsäure,
R-2-(4-Dimethylaminobiphenylsulfonyl)-1,2,3,4-tetrahydroisochinolin-3-carbonsäure,
R-2-(4-Benzoylphenylsulfonyl)-1,2,3,4-tetrahydroisochinolin-3-hydroxamsäure,
R-2-(4-Methoxybenzolsulfonyl)-7-hydroxy-1,2,3,4-tetrahydroisochinolin-3-hydroxamsäure,
R-2-(4-Methoxybenzolsulfonyl)-7-nitro-1,2,3,4-tetrahydroisochinolin-3-hydroxamsäure,
2-(4-Methoxybenzolsulfonyl)-6,7-propylen-1,2,3,4-tetrahydroisochinolin-1-hydroxamsäure,
R-5-(4-Methoxybenzolsulfonyl)-4,5,6,7-tetrahydro-1H-imidazo-(4,5-c)-pyridin-6-hydroxamsäure,
R-2-(4-Methoxybenzolsulfonyl)-1,2,3,4-tetrahydro-9H-pyrido-(3,4-c)-indol-3-hydroxamsäure oder
R-2-(4-Phenoxybenzolsulfonyl)-1,2,3,4-tetrahydro-9H-pyrido-(3,4-c)-indol-3-hydroxamsäure einsetzt.

Die Erfindung betrifft ferner Arzneimittel, enthaltend eine wirksame Menge von mindestens einer Verbindung der Formel I, wobei das zentrale Kohlenstoffatom zwischen Amino- und Hydroxamsäuregruppe als R-Enantiomer vorliegt.

Aufgrund der pharmakologischen Eigenschaften eignen sich die erfindungsgemäßen Verbindungen zur Prophylaxe und Therapie all solcher Erkrankungen, an deren Verlauf eine verstärkte Aktivität von Matrix-abbauenden Metalloproteinasen beteiligt ist. Dazu gehören degenerative Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen. Ferner gehören dazu auch Erkrankungen des Bindegewebes wie Kollagenosen, Periodontalerkrankungen, Wundheilungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels. Ferner eignen sich die Verbindungen der Formel I zur Behandlung der Ulceration, Atherosklerose und Stenosen. Weiterhin unterdrücken die Verbindungen der Formel I deutlich die Freisetzung des zellulären Tumor Nekrose Faktors (TNFα) und eignen sich daher zur Behandlung von Entzündungen, Krebserkrankungen, Tumormetastasenbildung, Kachexie, Anorexie und septischem Schock.

Die erfindungsgemäßen Arzneimittel werden im allgemeinen oral oder parenteral verabreicht. Die rektale oder transdermale Applikation ist auch möglich.

Die Erfindung betrifft auch ein Verfahren zur Herstellung eines Arzneimittels, das dadurch gekennzeichnet ist, daß man mindestens eine Verbindung der Formel mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel und Lösungsvermittler, Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylenglykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind - je nach Wirksamkeit der Verbindungen gemäß Formel 1, Tagesdosen von etwa 20 mg bis 1000 mg Wirkstoff, bevorzugt etwa 100 mg bis 500 mg indiziert. Unter Umständen können jedoch auch höhere oder niedrigere Tagesdosen angebracht sein. Die Verabreichung derTagesdosis kann sowohl durch Einmalgabe in Form einereinzelnen Dosierungseinheit oder aber mehrerer kleinerer Dosierungseinheiten als auch durch Mehrfachgabe unterteilter Dosen in bestimmten Intervallen erfolgen.

¹H-NMR-Spektren sind an einem 200-MHz-Gerät der Firma Varian aufgenommen worden, in der Regel mit Tetramethylsilan (TMS) als internem Standard und bei Raumtemperatur (RT). Die verwendeten Lösemittel sind jeweils angegeben. Endprodukte werden in der Regel durch massenspektroskopische Methoden (FAB-, ESI-MS) bestimmt. Temperaturangaben in Grad Celsius, RT bedeutet Raumtemperatur (22-26°C). Verwendete Abkürzungen sind entweder erläutert oder entsprechen den üblichen Konventionen.

### Herstellungsbeispiele

Die Herstellung der Verbindungen 1-12, 14-23, 27, 30 und 33 in Tabelle 1 erfolgte analog zu den in den Beispielen 13, 24-26, 28, 29, 31 und 32 dargestellten Verfahrensweisen.

Bei den Beispielen 4 bis 9 erfolgte zunächst eine Sulfonierung mit p-(Beisp. 4, 6, 9) bzw. m-(Beisp. 5,7,8) Nitrobenzolsulfonylchlorid wie unter "Tic-Sulfonierung" (siehe Beispiel 13) beschrieben. Anschließend erfolgt die Hydrierung der Nitrogruppe unter Standardbedingungen, die dem Fachmann bekannt sind, mit Wasserstoff unter Normaldruck und 10 % Pd auf Aktivkohle in Methanol zum Amin.
In allen Fällen ist es ebenso möglich, den unter Beispiel 13 beschriebenen Tic-Benzylester zur Sulfonierung einzusetzen. Bei der nachfolgenden Hydrierung erfolgt dann gleichzeitig Abspaltung des Benzylesters und Reduktion zum Amin. Die in beiden Fällen erhaltenen identischen Produkte p- bzw. m-Aminobenzolsulfonyl-Tic werden anschließend wie folgt weiter umgesetzt:

### Beispiel 4:

Es erfolgt zunächst Acetylierung unter Standardbedingungen (Triethylamin/DMAP/Acetanhydrid); die in guter Ausbeute erhaltene N-Acetyl-Verbindung wird anschließend wie unter Beispiel 25 beschrieben zur Hydroxamsäure weiter umgesetzt.

### Beispiel 5 und 6:

Für die Hydroxamsäure-Darstellung wird zur Aktivierung des p-Aminobenzolsulfonyl-Tic in gleicher Weise wie unter Beispiel 13 beschrieben verfahren, nur daß die doppelte Menge Chlorameisensäureethylester und N-Methylmorpholin eingesetzt wird. Es erfolgt irreversible N-Ethoxycarbonylierung in einem Schritt mit der Aktivierung der Carbonsäure.

### Beispiele 7, 8 und 9:

Das oben beschriebene p- bzw. m-Aminobenzolsulfonyl-Tic wird unter den dem Fachmann bekannten Schotten-Baumann-Bedingungen acyliert. Hierzu werden eingesetzt: Beisp. 7: Salicylsäurechlorid-, Beispiel 8: p-Methoxybenzoesäurechlorid, Beispiel 9: Chlorameisensäurebenzylester. Die weitere Umsetzung zur Hydroxamsäure erfolgt wie unter Beispiel 25 beschrieben.

### Beispiel 13:

### R-2-(4-Phenoxybenzolsulfonyl)-1,2,3,4-tetrahydroisochinolin-3-hydroxamsäure

### Allgemeine Arbeitsvorschrift:

### Tic-Benzylester p-Toluolsulfonat

1 Mol Tic (freie Aminosäure), 10 Mol Benzylalkohol und 1 Mol p-Toluolsulfonsäure Monohydrat werden in 1,2 1 Toluol gelöst oder suspendiert und am Wasserabscheider unter Rückfluß erhitzt. Nach Beendigung der Reaktion wird das Lösemittel abgedampft, der feste, kristalline Rückstand mehrfach in Diethylether aufgenommen und abgesaugt und anschließend im Ölpumpenvakuum getrocknet. Ausbeute: quantitativ
¹H-NMR: (200 MHz, δ in ppm, DMSO-d₆) 9,7 (s, br., 2 H, prot.NH), 7,5-7,25 (2m, 7H, arom.), 7,1 (d, 2H, arom. p-TsOH), 5,3 (s, 2H, CH₂ Benzyl); 4,7 (dd, 1 H, CHα); 4,4 "d", 2H, CH₂); 3,4-3,1 (m, 2H, CH₂); 2,3 (s, 1 H, CH₃ p-TsOH).

### Tic-Sulfonierung

0,1 Mol Tic-Lösung (freie Aminosäure 17,7 g) in 50 ml 2 N wäßriger NaOH wird bei 0°C mit fein gepulverten Sulfonsäurechlorid (105 mmol) versetzt, gefolgt von 14,2 g (110 mMol) Diisopropylethylamin und 50 ml Aceton oder THF. Nach 10 min wird das Eisbad entfernt und die mehr oder weniger homogene Lösung noch 6 h bei RT gerührt. Anschließend wird die Reaktionsmischung eingeengt, mit 300 ml Essigester versetzt und mit 4 N HCl angesäuert Die organische Phase wird abgetrennt, die wäßrige Phase wird noch zweimal mit je 50 ml Essigester extrahiert. Die vereinigten organischen Phasen werden mit gesättigter NaCl-Lösung ausgeschüttelt und über Natriumsulfat getrocknet. Nach dem Abdestillieren des Lösemittels verbleibt die sulfonierte Tetrahydroisochinolincarbonsäure als öliger oder fester Rückstand, der in manchen Fällen durch Umkristallisation aus Essigester/Petrolether gereinigt werden kann, oft aber auch schon hinreichend rein für die weitere Umsetzung ist.

### 13a R-2-(4-Phenoxybenzolsulfonyl)-1,2,3,4-tetrahydroisochinolin-3-carbonsäuremethylester

Eine Lösung von 1,92 g (0,01 Mol) R-1,2,3,4-Tetrahydroisochinolin-3-carbonsäuremethylester und 2,7 g (0,01 Mol) 4-Phenoxybenzolsulfonsäurechlorid in 50 ml absolutem THF werden in Gegenwart von 1,7 ml (0,01 Mol) N-Ethylmorpholin 8 h am Rückfluß erhitzt. Nach Entfernen des Lösungsmittels wird der Rückstand im Dichlormethan aufgenommen und nacheinander mit 5% Citronensäure, 5% Natriumbicarbonatlösung und 2 x mit Wasser ausgeschüttelt. Trocknen über Natriumsulfat und Einengen der organischen Phase ergibt den Ester, der ohne Reinigung weiterumgesetzt wird. Ausbeute: 4,0 g (95% der Theorie) an 13a.

### 13b R-2-(4-Phenoxybenzolsulfonyl)-1,2,3,4-tetrahydroisochinolin-3-carbonsäure

Eine Lösung von 4,0 g (9,5 mMol) des Esters (13a) in 50 ml iso-Propanol werden nach Zusatz von 9,5 ml 1N Natronlauge 24 h bei Raumtemperatur gerührt. Danach wird mit 1 n-Salzsäure angesäuert und die Mischung unter vermindertem Druck zur Trockenen eingeengt. Der Rückstand wird in Toluol aufgenommen, mit 5% Citronensäure ausgeschüttelt und nach Trocknen der organischen Phase über Natriumsulfat im Vakuum eingedampft.
Ausbeute: 3,4 g Carbonsäure 13b (83% der Theorie)
Schmelzpunkt: 147°C

### 13c R-2-(4-Phenoxybenzolsulfonyl)-1,2,3,4-tetrahydroisochinolin-3-hydroxamsäure

3,4 g (8,3 mMol) der Carbonsäure 13b werden in 30 ml DMF gelöst und bei -20°C nacheinander mit 1,4 g (12 mMol) N-Ethylmorpholin und 1,13 g (8,3 mmol) Chlorameisensäureisobutylesterversetzt. Nach einerAktivierungszeit von 30 min wird mit 4,37 g (41,5 mmol) O-Trimethylsilylhydroxylamin versetzt und für 4 h bei RT weitergerührt. Nach Zugabe von 250 ml Essigester und 500 ml Wasser wird mit Citronensäure angesäuert. Nach Abtrennen der organischen Phase und 4 x Ausschütteln der wäßrigen Phase werden die vereinigten organischen Phasen über Natriumsulfat getrocknet und unter vermindertem Druck eingeengt. Umkristallisation aus Toluol/Essigester (1:1) ergibt die Titelverbindung 13.
Ausbeute: 2,9 g (82% der Theorie) Schmelzpunkt: 170°C (Zersetzung)

### Beispiel 17:

Zur Sulfonierung, des Tic-Benzylesters unter Standardbedingungen (siehe Beispiel 13) wird trans-beta-Styrolsulfonylchlorid eingesetzt. Bei der nachfolgenden Hydrierung (H2, Pd/C) erfolgt in einem Schritt Debenzylierung und Hydrierung der Doppelbindung. Anschließend Hydroxamsäurebildung analog Beispiel 25.

### Beispiele 20, 21 und 22:

Ausgegangen wird von käuflich erhältlichen 7-Hydroxy-Tic. Dieses wird unter Standardbedingungen analog Verfahrensvariante d) sulfoniert. Hierbei erhält man, nach üblicher Aufarbeitung, eine Mischung des 2- und 7-disulfonierten sowie ausschließlich 2-sulfonierten-7-Hydroxy-Tic. Auf dieser Stufe kann allerdings auf eine Auftrennung beider Verbindungen verzichtet werden. Es erfolgt direkt weitere Umsetzung zur Hydroxamsäure unter Standardbedingungen. Wie erwartet kommt es während der Aktivierung dabei zu partieller Ethoxycarbonylierung der 7-Hydroxygruppe. Die Hydroxamsäure-Produktmischung enthält darum alle drei Produkte, die durch Chromatographie an Kieselgel 60, präparativer Dünnschichtchromatographie oder HPLC getrennt werden können.

### Beispiel 23:

Zur Darstellung des 7-Nitro-Tic wird von enantiomerenreinem käuflichen (R)-Tic-OH oder (S)-Tic-OH ausgegangen. Dieses wird nach E. D. Bergann, J. Am. Chem. Soc. 74, 4947 (1952) bzw. nach E. Erlenmever, A. Lipp, Liebigs Ann. Chem. 219, 218 (1983) durch Nitrierung mit Nitriersäure hergestellt. Dabei entsteht eine Mischung der 6- und 7-Nitro-Isomeren, wobei außerdem auch noch nicht nitrierte Ausgangsverbindung in der Reaktionsmischung enthalten ist. Vor der Auftrennung wird zunächst eine Sulfonierung des Gemisches unter Standardbedingungen durchgeführt. Die erhaltene Mischung der drei Sulfonamide kann nun an Kieselgel 60 chromatographiert werden. Man erhält nacheinander Mischfraktionen, die Edukt/6-Nitro- und 6-Nitro-/7-Nitro-(4-methoxybenzolsulfonyl)-Tic enthalten; zuletzt werden Reinfraktionen der 7-Nitro-Verbindung eluiert. Diese kann wie üblich analog Beispiel 25 weiter zur Hydroxamsäure umgesetzt werden.

### Beispiel 24:

### 2-(4-Methoxybenzolsulfonyl)-6,7-methylendioxy-1,2,3,4-tetrahydroisochinolin-3-hydroxamsäure

Die Herstellung des entsprechenden Carbonsäurebenzylesters aus der Carbonsäure entspricht der allgemeinen Arbeitsvorschrift (siehe Beispiel 13). Zur Sulfonierung oder Benzylesterspaltung wird analog Beispiel 25a verfahren. Die Umsetzung der freien, sulfonierten Carbonsäure erfolgt wie unter 25b beschrieben.
Das Produktfällt nach der Behandlung mit Diethylether kristallin an. Ausbeute: 140 mg, 57% d. Theorie; Schmelzpunkt 166°C.

### Beispiel 25:

### 2-(4-Methoxybenzolsulfonyl)-6,7,8-trimethoxy-1,2,3,4-tetrahydroisochinolin-3-hydroxamsäure

### 25a 2-(4-Methoxybenzolsulfonyl)-6,7,8-trimethoxy-1,2,3,4-tetrahydroisochinolin-3-carbonsäure

Die Herstellung des Benzylesters erfolgt nach der allgemeinen Arbeitsvorschrift (siehe Beispiel 13). Zur Sulfonierung werden 1,2 g (3,05 mMol) des Benzylesters eingesetzt. Dieser wird in 20 ml THF gelöst und bei 0°C mit 0,63 g (3,05 mMol) 4-Methoxybenzolsulfonsäurechlorid versetzt. Nach der Zugabe von 0,32 ml N-Methylmorpholin wird bei 0°C bis Raumtemperatur über Nacht gerührt. Anschließend wird mit 20 ml Essigester versetzt und mit 10 prozentiger Natriumcarbonatlösung sowie gesättigter NaCl-Lösung ausgeschüttelt. Die organische Phase wird über Natriumsulfat getrocknet, filtriert und unter vermindertem Druck eingedampft. Der verbleibende Rückstand wird an Kieselgel 60 mit Essigester/Petrolester/Eisessig 20/10/1 unter Druck chromatographiert. Reine Produktfraktionen (600 mg) werden vereinigt und nach dem Einengen direkt mit 100 mg 10% Pd/C in 50 ml Ethanol hydriert. Nach Beendigung der Reaktion wird der Katalysator abgetrennt und die verbleibende Lösung unter vermindertem Druck eingedampft. Man erhält 330 mg (66 % der Theorie).

### 25b 2-(4-Methoxybenzolsulfonyl)-6,7,8-trimethoxy-1,2,3,4-tetrahydroisochinolin-3-hydroxamsäure

330 mg (0,75 mMol) der Carbonsäure aus Beispiel 25a werden in 15 ml THF gelöst und bei -20°C nacheinander mit 0,07 ml (0,75 mMol) Chlorameisensäureethylester und 0,15 ml (1,5 mMol) N-Methylmorpholin (NMM) versetzt. Nach 30 min bei dieser Temperatur wird mit 0,474 ml O-Trimethylsilylhydroxylamin (3,75 mMol) versetzt. Nach 6 h bei RT wird 30 ml Essigester zugegeben und mit 20 prozentiger wäßriger Citronensäure sowie gesättigter NaCl-Lösung ausgeschüttelt. Nach dem Trocknen der organischen Phase über Natriumsulfat und Eindampfen unter vermindertem Druck verbleiben 290 mg eines hellen, viskosen Öls, das durch Behandlung mit Diethylether kristallin wird.

### Beispiel 26:

### 2-(Morpholinosulfonyl)-1,2,3,4-tetrahydroisochinolin-3-hydroxamsäure

### 26a 2-(Morpholinosulfonyl)-1,2,3,4-tetrahydroisochinolin-3-carbonsäuremethylester

Zu einer Lösung von 4,8 g (0,025 Mol) 1,2,3,4-Tetrahydroisochinolin-3-carbonsäuremethylester und 2,9 g (0,025 Mol) N-Ethylmorpholin werden unter Rühren 4,2 g (0,025 Mol) Morpholin-N-sulfonsäurechlorid in 20 ml THF zugetropft. Nach 2 h Rühren bei RT wird zur Vervollständigung der Reaktion nach 2 h am Rückfluß erhitzt. Nach Behandlung der mit CHCl₃ angereicherten Reaktionslösung mit 5 prozentiger Citronensäure, 5 prozentiger NaHCO₃₋Lösung und Wasser, wird die organische Phase über Na₂SO₄ getrocknet und zur Trocknen eingeengt. Ausbeute an Ester (26a): 7,5 g (92% der Theorie)

### 26b 2-(Morpholinosulfonyl)-1,2,3,4-tetrahydroisochinolin-3-carbonsäure Umsetzung von 7,5 g (0,023 Mol) 26a analog 13b.

### Ausbeute an Carbonsäure 26b : 6,7 g (93% der Theorie)

26c2-(Morpholinosulfonyl)-1,2,3,4-tetrahydroisochinolin-3-hydroxamsäure2,3g(7,5mMol)derCarbonsäure 26b werden in 40 ml absoluten THF gelöst und bei -20°C nacheinander mit 1,2 g (12 mMol) N-Methylmorpholin und 1,1 g (7,5 mMol) Chlorameisensäureisobutylester versetzt. Nach 30 min wird mit 3,9 g (37,5 mMol) O-Trimethylsilylhydroxylamin versetzt und weitere 5 h bei RT gerührt. Nach Zugabe von 200 ml Wasser wird mit verdünnter HCL angesäuert und mit Dichlormethan mehrmals ausgeschüttelt. Die gesammelten organischen Phasen werden über Na₂SO₄ getrocknet und unter vermindertem Druck eingedampft. Das erhaltene Öl wird an Kieselgel 60 unter Druck mit Essigester/Dichlormethan (1:1) als mobile Phase chromatographiert.

Umkristallisation der Produktfraktionen aus Essigester ergab kristalline Hydroxamsäure 26c.
Ausbeute: 1,4 g (55% der Theorie) Schmelzpunkt: 164-165°C (Zersetzung)

### Beispiel 28:

### 1-(4-Methoxybenzolsulfonyl)-indolin-2-hydroxamsäure

### 28a 1-(4-Methoxybenzolsulfonyl)-indolin-2-carbonsäure

1 g (6,1 mMol) Indolin-2-carbonsäure und 2,5 g (12,2 mMol) 4-Methoxybenzolsuwonylchlorid werden bei 50°C und 0,02 mbarfür4 Stunden (h) im Kugelrohr unter ständigem langsamen Drehen belassen. Das bräunliche, kristalline Produkt wird anschließend in Natriumcarbonat-Lösung aufgenommen und zweimal mit Diethylether ausgeschüttelt. Die wäßrige Phase wird mit 6 N HCI angesäuert und viermal mit Essigester extrahiert. Die vereinigten organischen Phasen werden nach dem Ausschütteln mit gesättigter NaCl-Lösung über Natriumsulfat getrocknet und unter vermindertem Druck eingedampft. Verbliebene Lösemittelreste werden im Ölpumpenvakuum entfernt. Ausbeute: 1,34 g, (65 % der Theorie)
¹H-NMR: (DMSO-d₆) 7,8; 7,1 (2d, 4H, arom. p-TsOH); 7,4-7,0 (m, 4H, arom.); 4,9 (dd, 1H, CHα); 3,8 (2, 3H, OMe); 3,4-2,9 (2 dd, 2H, CH₂)

### 28b 1-(4-Methoxybenzolsulfonyl)-indolin-2-hydroxamsäure

1,3 g (3,9 mmol) der 1-(4-Methoxybenzolsulfonyl)-indolin-2-carbonsäure gemäß Beispiel 28a werden in 10 ml N,N-Dimethylacetamid (DMA) gelöst und bei -20°C nacheinander mit 0,37 ml (1 Äquivalent) Chlorameisensäureethylester und 0,81 ml N-Methylmorpholin versetzt. Nach einer Aktivierungszeit von 30 Minuten (min) wird mit 3,8 ml (19,5 mmol) O-Trimethylsilylhydroxylamin versetzt und für 4 h bei RT weitergerührt. Nach dem Verdünnen mit Essigsäureethylesterwird mit Citronensäure angesäuert und nach dem Abtrennen der wäßrigen Phase mit gesättigter NaCl-Lösung gewaschen. Die organische Phase wird über Natriumsulfat getrocknet, abfiltriert und unter vermindertem Druck eingedampft. Das erhaltene Öl wird an Kieselgel 60 unter Druck mit Dichlormethan/Essigsäureethylester/Essigsäure 5,5/3,5/1 als mobiler Phase chromatographiert. Produktfraktionen (mit positiver Eisen-III-chlorid-Reaktion) werden vereinigt und eingedampft. Das kristalline Produkt wird anschließend mit Diethylether versetzt und unter vermindertem Druck von Lösemittelresten befreit. Ausbeute: 400 mg (33 % der Theorie) Schmelzpunkt: 142°C

### Beispiel 29:

### R-5-(4-Methoxybenzolsulfonyl)-4,5,6,7-terahydro-1 H-imidazo-(4,5-c) pyridin-6-hydroxamsäure-hydrochlorid

### 29a: R-3,5-Di(4-methoxybenzolsulfonyl)-4,5,6,7-tetrahydro-1H-imidazo-(4,5-c) pyridin-6-carbonsäure

Zu einer Lösung von 6,1 g (30 mMol) 4,5,6,7-Tetrahydro-1H-imidazo-(4,5-c) pyridin-6-carbonsäure-hydrochlorid in 50 ml Wasser werden unter Eiskühlung nacheinander 15 ml 2N-NaOH und 4,5 g (42 mMol) Natriumcarbonat gegeben. Unter Rühren erfolgt die Zugabe von 13,7g (67 mMol) 4-Methoxybenzolsulfonylchlorid in 40 ml Ether. Nach 24 Stunden weiteren Rührens bei RT wird das Reaktionsgemisch unter Eiskühlung mit 5N-HCL auf pH 3-4 gestellt und mit Essigester extrahiert. Die organische Phase ergibt nach Trocknen über Natriumsulfat, Filtrieren und Einengen zur Trockenen 11,9 g (78% der Theorie) des gewünschten Produkts in Form eines Öls.

### 29 b: R-5-(4- Methoxybenzolsulfonyl)-4,5,6,7-tetrahydro-1H-imidazo-(4,5-c) pyridin-6-carbonsäure-hydrochlorid

Zu einer Lösung von 11,0 g (24 mMol) Di-sulfonierten Zwischenprodukes in 300 ml Methanol werden unter Eiskühlung im Abstand von 1 Stunde jeweils 23,5 ml einer 1 N-NaOH unter Rühren zugetropft. Nach 6 Stunden erfolgt abschließende Zugabe von 15 ml 1N-NaOH und Weiterrühren bei RT über Nacht. Nach Entfernen des Methanols im Vakuum, wird das Gemisch mit 5N-HCL auf pH 5 eingestellt. Die dabei ausfallenden Kristalle werden abgesaugt und über P205 im Vakuum getrocknet.
Ausbeute: 5,2 g (60 % der Theorie) an 29 b
Schmelzpunkt: 264- 265°C (Zers.)

### 29 c: R-5-(4- Methoxybenzolsulfonyl)-4,5,6,7-tetrahydro-1H-imidazo-(4,5-c)-pyridin-6-hydroxamsäure-hydrochlorid

8,0 g (24 mMol) Verbindung 29b in 60 ml DMF werden mit 4,27 g (24 mMol) Tetramethylammoniumhydroxyd und danach bei 0°C mit 2,7 g (24 mMol) N-Etylmorpholin und portionsweise mit 5,2 g (24 mMol) Di-tert.-butyldicarbonat versetzt. Nach Rühren über Nacht wird die Reaktionsmischung auf Eiswasser gegossen, mit verdünnter HCl auf pH 5 eingestellt und mehrmals mit Ethylacetat ausgeschüttelt. Die vereinigte, getrocknete organische Phase ergibt nach Entfernen des Lösemittels 10,5 g BOC-geschütztes 29b, das direkt zur Darstellung der Hydroxamsäure verwendet wird. Dazu wird 10,5 g (23 mmol) obiger Verbindung in 150 ml absolutes THF gelöst, und bei -20°C mit 4,4 g (38 mMol) N-Ethylmorpholin und 3,4 g (25 mMol) Isobutylchlorformiat versetzt. Nach 1 Stunde Rühren erfolgt die Zugabe von 10,9 g (0,1 Mol) O-Trimethylsilylhydroxylamin, wobei die Temperatur über 1 Stunde auf -20°C gehalten wird. Nach weiteren 4 Stunden Rühren bei RT wird die Reaktionsmischung mit 1 N-HCl auf pH = 1 eingestellt, mit 300 ml Wasser versetzt, und mehrmals mit Dichlormethan extrahiert. Die vereinigten organischen Phasen werden über Natriumsulfat getrocknet und im Vakuum zur Trockenen eingeengt.
Zur Abspaltung der BOC-Schutzgruppe werden 8,1 g des verbleibenden Öls in 50 ml Dichlormethan aufgenommen und bei 0°C 25 ml Trifluoressigsäure zugetropft. Nach 4 Stunden Rühren bei RTwird das Reaktionsgemisch im Vakuum eingeengt. Der Rückstand wird mit Dichlormethan digeriert, danach in 0,1 N-HCl gelöst, filtriert und gefriergetrocknet. Ausbeute an Hydroxamsäure 29 : 5,2 g (56% der Theorie)
Schmelzpunkt: 110°C

### Beispiel 31:

### R-2-(4-Methoxybenzolsulfonyl)-1,2,3,4-tetrahydro-9H-pyrido-(3,4-b)-indol-3-hydroxamsäure

### 31 a R-2-(4-Methoxybenzolsulfonyl)-1,2,3,4-tetrahydro-9H-pyrido(3,4-b)-indol-3-carbonsäure

Eine Lösung von 2,16 g (10 mMol) 1,2,3,4-Tetrahydro-9H-pyrido (3,4-b)-indol-3-carbonsäure in einem Gemisch von 10 ml Aceton und 10 ml Wasser wird nach Zugabe von 10,5 ml 2N NaOH unter Rühren mit 2,06 g (10 mmol) 4-Methoxybenzolsulfonylchlorid versetzt. Nach 18 stündigem Rühren bei Raumtemperatur wird nach dem Entfernen des Acetons die Lösung mit konzentrierter HCl auf pH =1 eingestellt. Der dabei entstehende Niederschlag wird abfiltriert, mit Wasser gewaschen und getrocknet.
Ausbeute: 2,7 g Carbonsäure 31 a (85% der Theorie)
Schmelzpunkt: 232-234°C

### 31 b R-2-(4-Methoxybenzolsulfonyl)-1,2,3,4-tetrahydro-9H-pyrido(3,4-b)-indol-3-hydroxamsäure

2,5 g (7,4 mMol) der Carbonsäure 31 a werden in 40 ml absolutem DMF gelöst und bei -20°C nacheinander mit 1,4 ml (12 mmol) N-Ethylmorpholin und 0,97 ml (7,4 mmol) Chlorameisensäureisobutylester versetzt. Nach einer Aktivierungszeit von 30 min wird 4,53 ml (37 mMol) O-Trimethylsilylhydroxylamin hinzugegeben und anschließend 19 Stunden bei Raumtemperatur gerührt. Nach Einstellung der Mischung auf pH= 3,5 mit Citronensäure wird mehrmals mit Essigester extrahiert. Die vereinigten organischen Phasen werden über Natriumsulfat getrocknet, unter vermindertem Druck eingeengt und über Kieselgel mit Methylenchlorid/Methanol (95:5) chromatographisch gereinigt. Ausbeute: 2,4 g Hydroxamsäure (91,5% der Theorie) Schmelzpunkt: 87°C

### Beispiel 32:

### R-2-(4-Phenoxybenzolsulfonyl)-1,2,3,4-tetrahydro-9H-pyrido(3,4-b)-indol-3-hydroxamsäure

Herstellung analog Beispiel 31
Schmelzpunkt: 110-111 °C

### Beispiel 33:

### R-2-(4-Morpholinobenzolsulfonyl)-1,2,3,4-tetrahydro-9H-pyrido(3,4-b)-indol-3-hydroxamsäure

Herstellung analog Beispiel 31
Schmelzpunkt: 125°C (Zersetzung)

### Beispiel 42:

### R-2-[4-(4-Chlorphenoxy)benzolsulfonyl)-1,2,3,4-tetrahydro-isochinolin-3-carbonsäure

8,2 g (46,4 mmol) R-1,2,3,4-Tetrahydroisochinolin-3-carbonsäure werden mit 46,4 ml 1 N NaOH und 50 ml Aceton versetzt und mit Wasser zur Lösung gebracht. Bei -5°C wird unter Rühren 14,1 g (46,4 mmol) 4-(4-Chlorphenyloxy)-benzolsulfon-säurechlorid in 50 ml THF zugetropft, wobei nach der Hälfte der Zugabe das Reaktionsgemisch mit 6,0 g (46,4 mmol) Düsopropylethylamin versetzt wird. Nach Rühren über Nacht wird vom Niederschlag abfiltriert, das Filtrat mit 2 N HCl auf pH=3 eingestellt und mehrmals mit Dichlormethan extrahiert. Die vereinten organischen Phasen werden, nach Trocknen über Natriumsulfat, filtriert und zur Trockenen unter vermindertem Druck eingedampft. Umkristallisation aus Toluol und Trocknen unter vermindertem Druck ergibt die Titelverbindung.
Ausbeute: 16,1 g (78 % der Theorie) Schmelzpunkt: 168-169°C

**Tabelle 1: Hydroxamsäuren der allgemeinen Formel I**

| Beispiel-Nr. | Struktur | Schmp. (°C) | Solvens | ¹H-NMR |
|---|---|---|---|---|
| 1 | 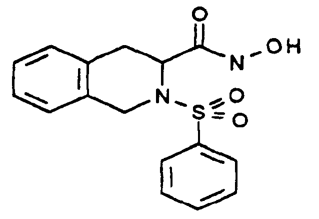 | | DMSO-d6 | 2,7-3,1 (m, 2 H) 4-4,7 (2 m, 2 H) 7-7,8 (3 m, 9 H) 9,5; 10,6 (2 s, br, 2 H) |
| 2 | 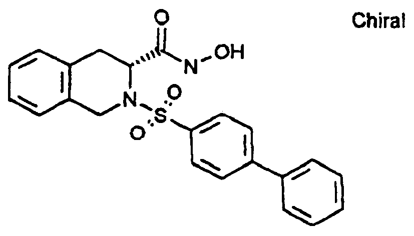 | 94 Zers. | CDCL3 | 2,65-2,8 (m, 1H) ; 3,1-3,25 (m, 1H); 4,35-4,75 (m, 3H); 6,9-7,2 (m. 4H); 7,3-7,65 (m, 7H); 7,8 (d,2H) |
| 3 | 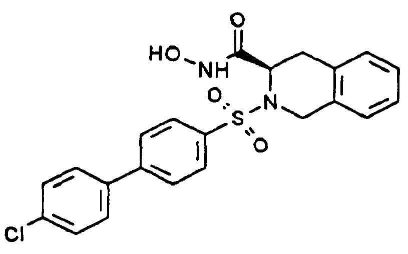 | | DMSO-d6 | 2,9 (m,2H); 4,5 (t, 1 H); 4,6(m,2H); 7,0-7,9(m12H); 9,9(s,1H);10,8 (s,1H) |
| 4 | 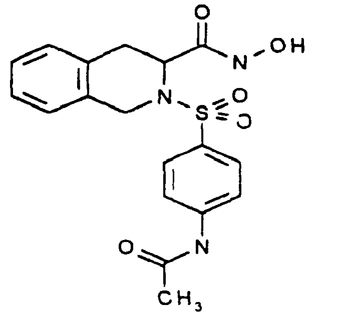 | | DMSO-d6 | 2,1 (s, 3 H) 2,8-3,5 (2 m, 2 H), 4,3-4,6 (m, 3 H) 7,1; 7,7 (2 m, 8 H) 8,65; 8,85; 10,3; 10,8 (4 s, 2 h) |
| 5 | 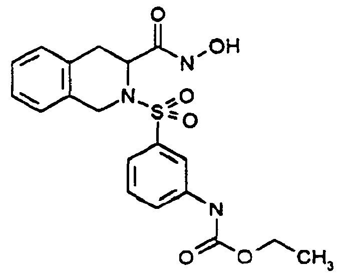 | | DMSO-d6 | 1,2 (t, 3 H) 2,85 (m, br, 2 H) 4,15 (q, 2 H) 4,4-4,7 (m, 3 H) 7,1 (m, br, 4 H) 7,4 (m, 2 H) 7,6 (m, 2 H) 8; 9,9; 10,7 (3 s, 3 H) |
| 6 | 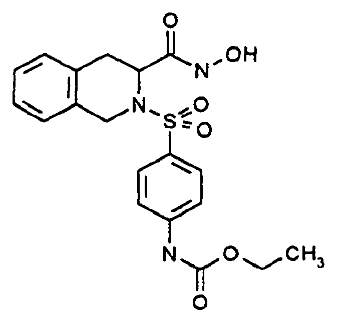 | | DMSO-d6 | 1,2 (t, 3 H) 2,8 (m, br, 2 H) 4,15 (q. 2 H) 4,3-4,6 (m, 3 H) 7,1 (m. br, 4 H) 7,55; 7,7 (2 d, 4 H) 8,7; 9,5 (2 s, 3 H) |
| 7 | 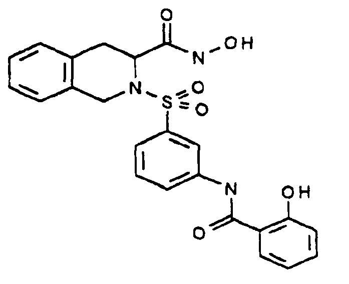 | | DMSO-d6 | 2 (s, 3 H) 2,9 (m, 2 H) 4,4-4,6 (2 m, 3 H) 7,1; 7,5; 7,9; 8,3 (4 m, 14 H) |
| 8 | 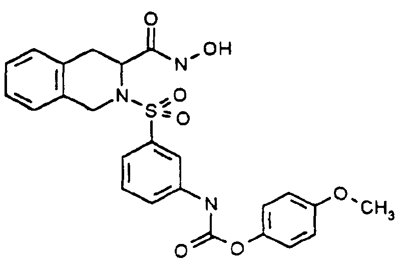 | | OMSO-d6 | 2,85 (m, 2 H) 3,85 (s, 3 H) 4,4-4,7 (2 m, 3 H) 7,1; 7,4; 7,6; 8 (4 m, 13 H) 8,9: 10,8 (2 s, 2 H) |
| 9 | 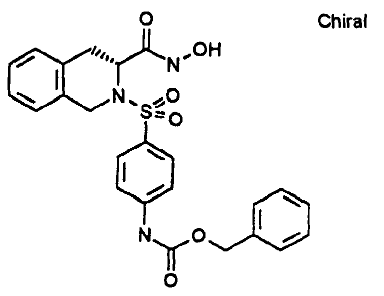 | | DMSO-d6 | 3 (m, 2H) 4,4-4,8 (m, 3 H) 5,2 (s, 3 H) 7,1-7,5 (2 m, 9 H) 7,55; 7,8 (2 d, 4 H) 8,8; 10,7 (2 s, 2 H) |
| 10 | 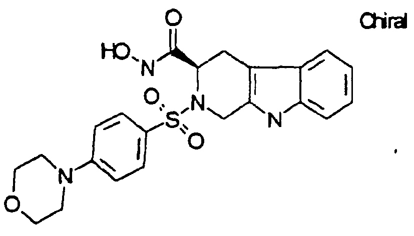 | 175 Zers. | DMSO-d6 | 2,7-3,0(m,2H); 3,25(m,4H); 3,75(m,4H); 4,45 (t, 1H); 4,5(M,2H); 6,9-7,65(m,8H) |
| 13c | 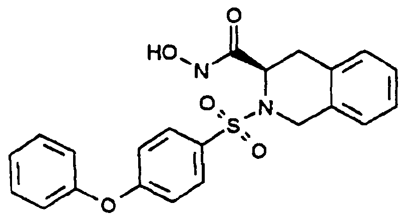 | 170 Zers. | DMSO-d6 | 2,9(d,2H); 4,4(m,2H); 4,55(d.1 H); 6,9-7,85(m,13H); 8,9(s,1H); 10,75(s,1H); |
| 14 | 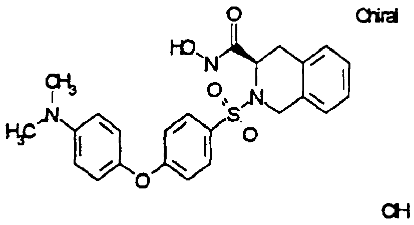 | | DMSO-d6 | 2,9(m,2H); 3,64(s6H); 4,3 8 (t, 1H); 4,5(m,2H); 6.75-7,75(m,12H); |
| 15 | 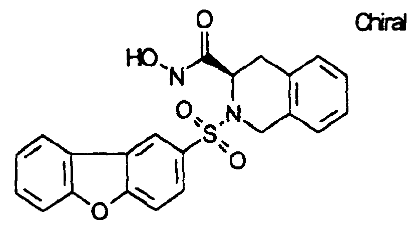 | | DMSO-d6 | 2,85(m,2H); 4,45(t,1 H); 4,63(m,2H); 6,9-8,7 (m, 11 H); 9,9(s,1 H); 10,8(s,1H); |
| 16 | 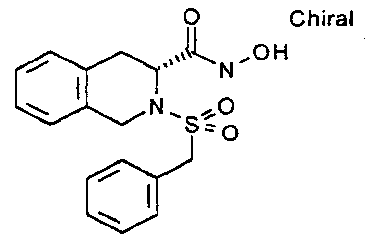 | | DMSO-d6 | 2,9-3,1 (m, 2 H) 3,9-4,6 (2 m, 5 H) 7.15 (m, 4 H) 7,3 (m, 5 H) 8,85; 10,6 (2 s, 2 H) |
| 17 | 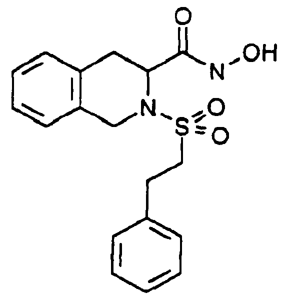 | | DMSO-d6 | 2,8-3,6 (m, 6 H) 4,5-4,7 (m, 3 H) 7,1-7,4 (m, 9 H) 8,7; 8,9; 9,5; 10,7 (4 s, 2 H) |
| 18 | 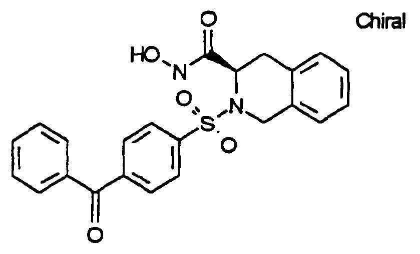 | | DMSO-d6 | 2,95(m,2H); 4,5(t,1 H); 4,62(m2H); 7,0-8,05(m,13H); |
| 19 | 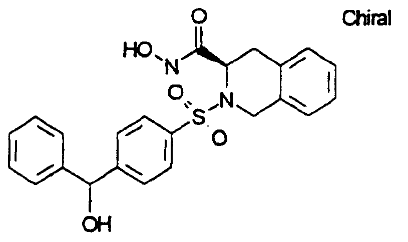 | | DMSO-d6 | 2,85(m,2H); 4,4(M,1 H); 4,53(m,2H); 6,95-7.8(M;13H); |
| 20 | 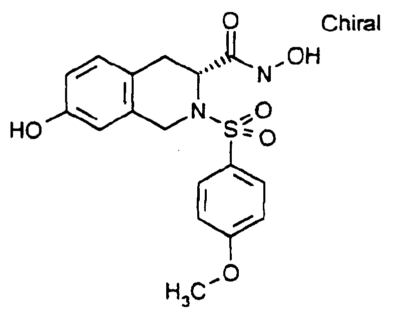 | | DMSO-d6 | 2,8 (m, 2 H) 3,8 (s, 3 H) 4,35-4,6 (m, 3 H) 6,9-7,2; 7,6-7,8 (2 m, 7 H) 8,9; 10,8 (2 s, 2 H) |
| 21 | 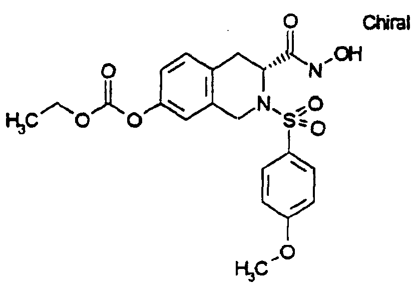 | | DMSO-d6 | 1,3 (t. 3 H) 2,85 (m, 2 H) 3,8 (s, 3 H) 4,0-4,6 (m, 5 H) 6,9-7,1; 7,6-7,8 (2 m, 7 H) 8,8; 10,8 (2 s, 2 H) |
| 22 | 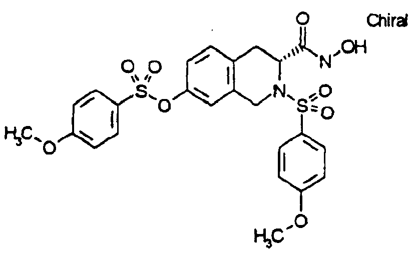 | | DMSO-d6 | 2,8 (m, 2 H) 3,8 (s, 3 H) 3,9 (s, 3 H) 4,35-4,6 (m, 3 H) 6,9-7,2; 7,6-7,8 (2 m, 11 H) 8,9; 10,9 (2 s, 2 H) |
| 23 | 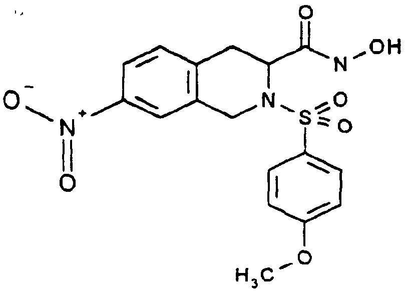 | | DMSO-d6 | 3,0 (m, 2 H) 3,8 (s, 3 H) 4,4-4,8 (m, 3 H) 6,95; 7,7 (2 d, 4 H) 7,4 (d, 1 H) 7,95 (dd, 1 H) 8,05 (d, 1 H) 8.95: 10,8 (2 s. 2 H) |
| 24 | 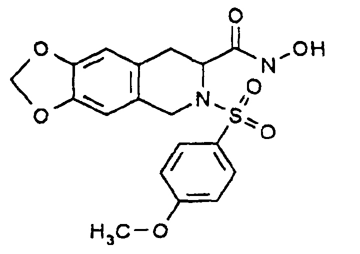 | 166 | DMSO-d6 | 2,7 (m, 2 H) 3,8 (s, 3 H) 4,2-4,5 (m, 3 H) 5,9; 6,7; 7,0; 7,7 (4 d, 6 H) 8,85; 10,7 (2 s, 2 H) |
| 25 | 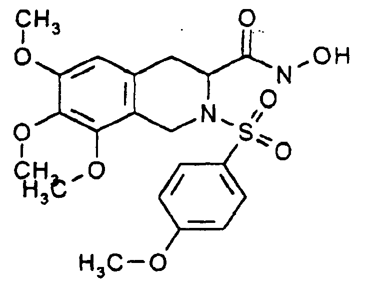 | | DMSO-d6 | 2,8 (m, 2 H) 3,65-3,85 (4s, 12 H) 4,3-4,5 (m, 3 H) 6,5 (s, 1 H) 7,0; 7,7 (2 d, 4 H) 8,8; 10,7 (2 s, 2 H) |
| 26 | 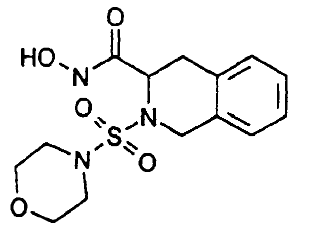 | 165 | DMSO-d6 | 2,9-3,35(m,6H); 3,45-3,65(m,4H); 4,38(m, 1H);4,5; 4,65(AB,2H); 7,2 (s,4H); 8,9(s,1H); 10,65(s, 1H) |
| 27 | 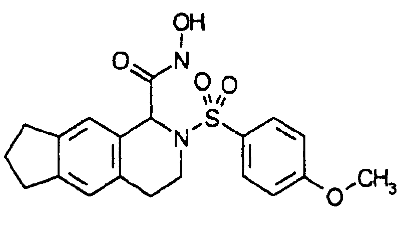 | | DMSO-d6 | 1,95 (m, 2 H); 2,5-2,95 m, 7 H); 3,4 (m, 1H); 3,8 (s,3 H); 3.8-4.1(m, 1 H); 6,9- 7.1(m. 4H);7,7 (d, 2 H); 9,0-11,1 (2 s, 2 H); |
| 28 | 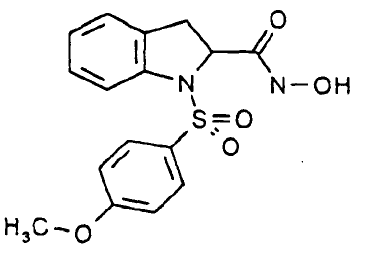 | 142 | DMSO-d6 | 2,8-3,2 (m, 2 H) 3,8 (s, 3 H) 4,6 (dd, 1 H) 7,0-7,8 (3 m, 8 H) 9,1; 10,9 (2 s, 2 H) |

**Tabelle 2: Carbonsäuren der allgemeinen Formel I**

| Bsp. | Struktur | Schmp. (°C) | ¹HMR |
|---|---|---|---|
| 34 | 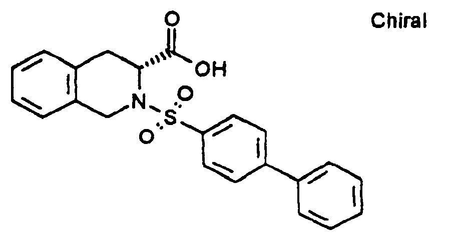 | 205 | (in CDCl3): 3,0-3,25 (m, 2H); 4,48 (d, 1 H); 4,65 (d, 1 H); 4,9-5,0 (m, 1H); 6,97-7,18 (m, 4H); 7,38-7,7 (m, 7H); 7,85 (d, 2H) |
| 35 | 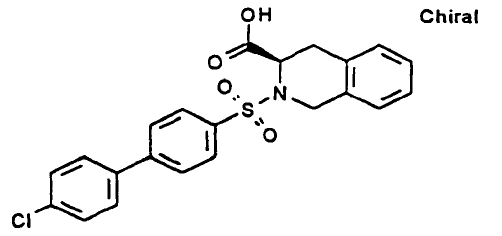 | 207-209 | (in DMSO-d6): 3,05-3,15(m,2H); 4,45-4,7(d,d,2H); 4,9(m,1H); 7,1-8,0 (m,12H); 12,8(s,1H); |
| 36 | 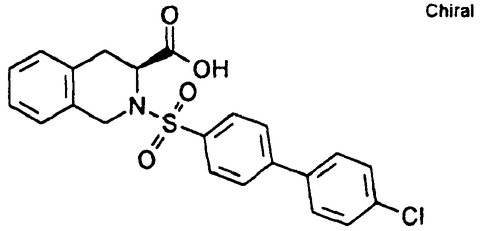 | | 3,1 (m, 2 H); 4,6 (m, 2 H); 4,90 (d, 1 H); 7,0-8,0 (2m, 12 H) |
| 37 | 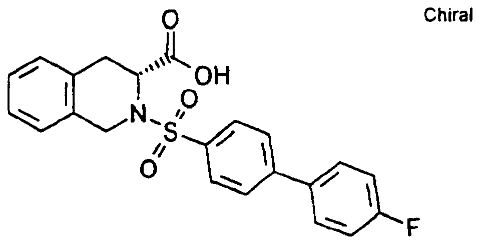 | | 3,0-3,2 (m, 2 H); 4,55 (dd, 2 H); 4,90 (d,1 H): 7,05-7,25 (m, 4 H); 7,1-8,0 (3 m. 12 H) |
| 38 | 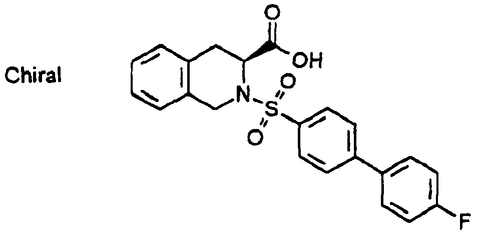 | | 3,0-3,2 (m, 2 H); 4,55 (dd, 2 H); 4,90 (d,1H); 7,05-7,25 (m, 4 H); 7,1-8,0 (3 m, 12 H) |
| 39 | 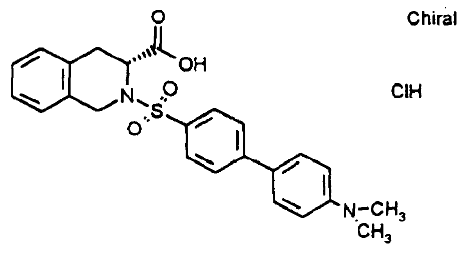 | 122-135 amorph | (in MeOH-d4): 3,02-3,36 (m, 2H u. s, 6H); 4,57 (d, 1H); 4,72 (d, 1H); 4,85-5,01 (m,1 H); 7,03-7,19 (m, 4H); 7,54 (d, 2H); 7,7-7,98 (m, 6H) |
| 40 | 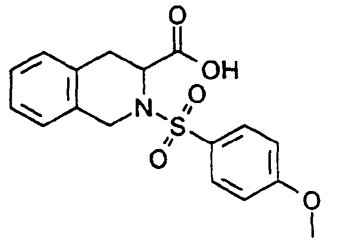 | | 2,9-3,2 (m, 2 H); 3,8 (s. 3 H); 4,3-4,6 (dd, 2 H); 4,8 (m, 1 H); 7,1 (m, 6 H); 7,8 (d, 2 H) |
| 41 13b | 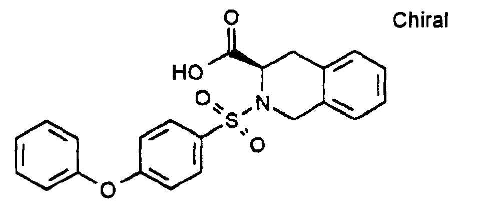 | 147 | (in DMSO-d6): 3,0-3,15(m,2H); 4,4-4,65(d,d,2H); 4,8-4,9 (m,1 H);7,0-7,9 (m, 13 H); 12,9(s,1 H); |
| 42 | 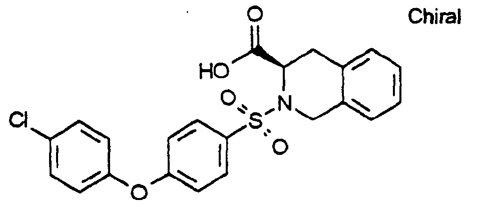 | 167-168 | (in DMSO-d6): 3,0-3,15(m2H); 4,4-4,65(m,2H); 4,85 (m,1H); 7,0-7,9 (m, 12 H); 12,9 (s, 1H); |
| 43 | 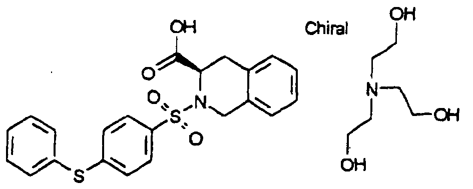 | Öl | (in DMSO-d6):2,4-2,7 (m, 6H) 2,8-3,0(m,2H); 3,3-3,5(m,6H); 4,4-4,6(m,2H); 4.7(m,1H); 7,0-7,9(m,13H) |
| 44 | 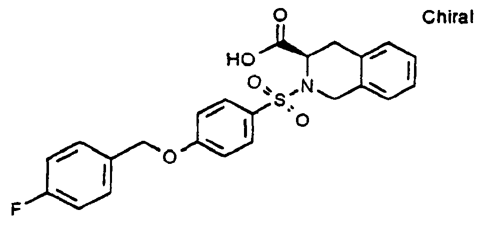 | | in DMSO-d6): 2,9-3,2(m,2H); 4,4-4,65 (d,d,2H); 4,85(m,1H); 5,15(s,2H); 7,0-7,9 (m,12H); 12,9(s,1H); |
| 45 | 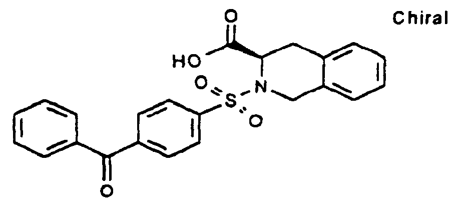 | Öl | (in DMSO-d6): 3,0-3,2 (m,2H);4,4-4,75(d,d, 2H); 4.9(m, 1H); 7,1-8,1 (m,13H); 12,9(s, 1 H); |
| 46 | 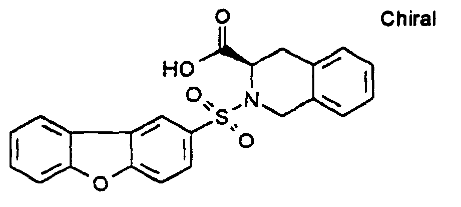 | 218-219 | (in DMSO-d6): 3,0-3,1 (m,2H); 4,45-4.8(d,d,2H); 4,9-5,0(m, 1H); 7,0-8,8(m, 11H); 12,8(s.1H); |
| 47 | 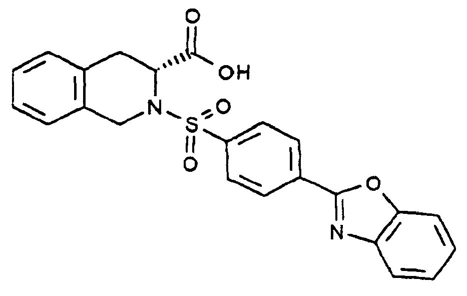 | 211-213 amorph | 3,0-3,2 (m, 2H); 4,5 (d, 1H); 4,72 (d, 1H); 4,9-5,05 (m, 1H); 7,05-7,25 (m, 4H); 7,6-7,75 (m, 3H); 7,85-8,05 (m, 2H); 8,2-8,4 (m; 3H); 12,9 (sb, 1 H) |
| 48 | 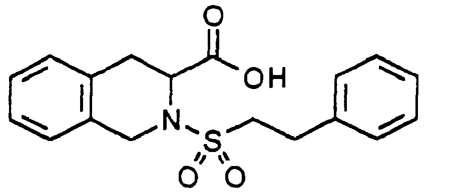 | | 3,0; 3,2 (2m, 4 H); 3,3-3,6 (m, 2 H); 4,5-4,75 (dd, 2 H); 4,8 ("t", 1 H); 7.1-7,4 (m, 9 H) |
| 49 | 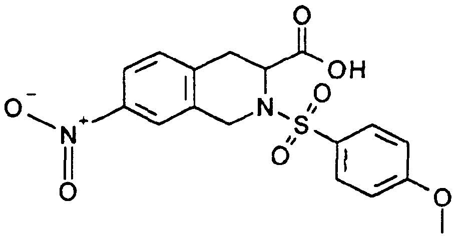 | | 3,0-3,3 (m, 2 H); 3,8 (s, 3 H); 4.45-4,B5 (dd, 2 H); 4,85 (m, 1 H); 7,0; 7,4; 7,8 (3 d, 5 H); 8,0 (dd, 1 H); 8,1 (d, 1 H) |
| 50 | 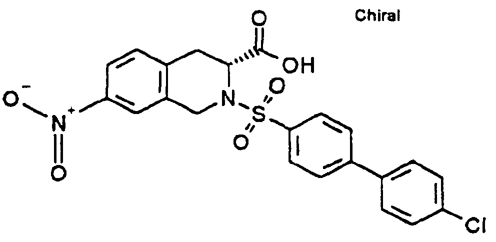 | | 3,3 (m, 2 H); 4,5-4,85 (dd, 2 H); 5,05 (m, 1 H); 7,2-8,1 (mm, 11 H) |
| 51 | 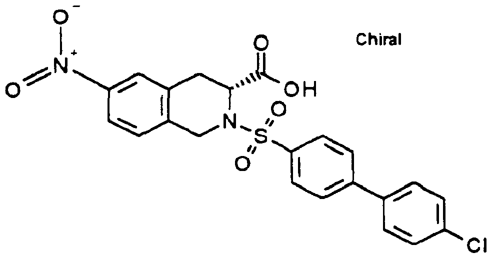 | | 3,3 (m, 2 H); 4,5-4,8 (dd, 2 H); 5,05 (dd, 1 H); 7,2-8,0 (4m, 11 H) |
| 52 | 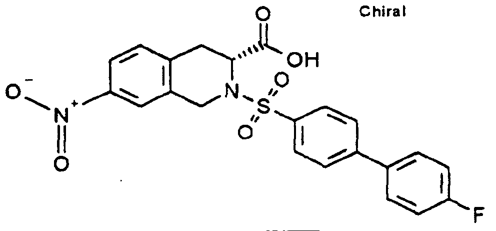 | | 3,1-3,4 (m, 2 H); 4,5-5,0 (dd, 2 H); 4,95 (m, 1 H); 7,2-8,1 (2m, 11 H) |
| 53 | 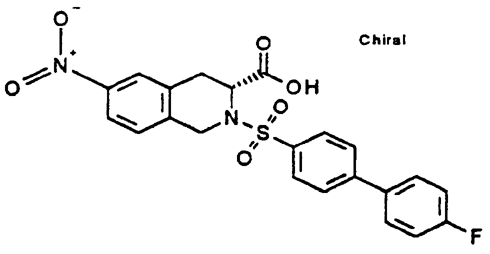 | | 3,1-3,4 (m, 2 H); 4,5-4,9 (dd, 2 H); 4,95 (m, 1 H); 7,2-8,15 (2m, 11 H) |
| 54 | 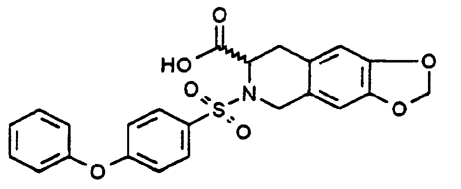 | 226-228 | (in DMSO-d6): 2,8-3,1(m,2H); 4,3-4,5 (d,d,2H); 4.75(m, 1H); 5,95(s,2H); 6,7-7,9 (m, 11H); 12,9(s,1 H); |
| 55 | 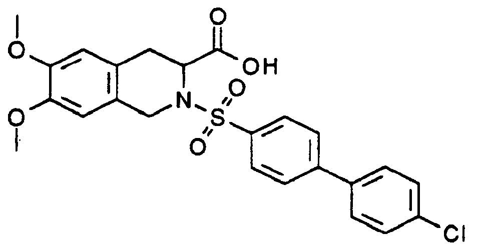 | | 2,9-3,1 (m, 2H); 3,8 (s, 6 H): 4,35 -4,6 (dd, 2H); 4,90 (d, 1 H); 6,7; 6,8 (2 s, 2 H); 7,55; 7,80 (2 d, 4H); 7,9 (m, 4 H) |
| 56 | 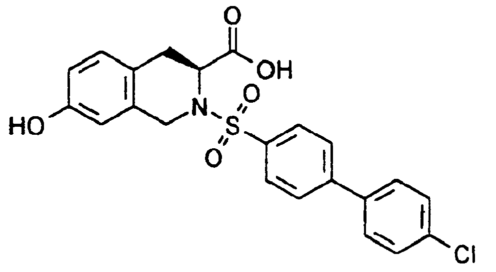 | | 2,8-3,1 (m, 2H); 4,3-4,6 (dd, 2H); 4,85 (m, 1H); 6,5 (m, 2 H); 6,95 (d, 1 H); 7,5-8,0 (m, 8 H); 8,5; 8,8 (2s, 1 H) |
| 57 | 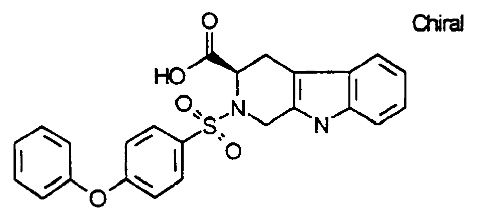 | 115 | (in DMSO-d6): 3,3-3,45(m,2H); 4,4-4,65 (m,2H); 5,8-5,9(m,1H); 6,85-7,9 (m,13H); 10, 7(s, 1H); |
| 58 | 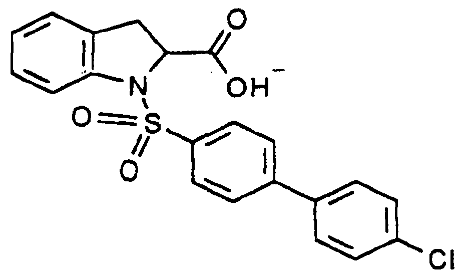 | | 3,1; 3,4 (2 m, 2 H); 5,05 (m, 1 H); 7,0-8,0 (m, 12 H) |
| 59 | 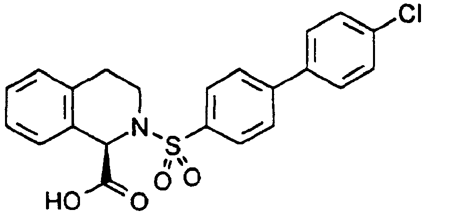 | | 2,8-3,0 (m, 2H); 3,5-3,8 (m, 2 H); 4,3 (s, 1H); 7,1-8,0 (mm, 12 H) |
| 60 | 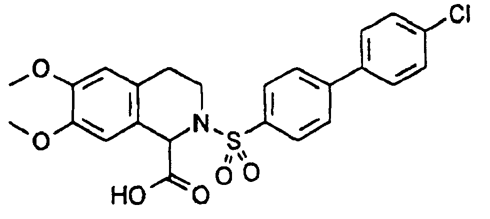 | | 2,7-2,9 (m, 2H); 3,4-3,8 (m, 2 H); 3,8 (2 s, 6 H); 5,4 (s, 1 H); 6,7; 6,9 (2 s, 2 H); 7,55; 7,80 (2 d, 4 H); 7,9 (s, 4 H) |

### Pharmakologische Beispiele

Darstellung und Bestimmung der enzymatischen Aktivität der katalytischen Domäne des humanen Stomelysins und der Neutrophilenkollagenase.

Die beiden Enzyme wurden dargestellt nach Ye et al., (Biochemistry 31(1992) 11231-5). Zur Messung der Enzymaktivität oder der Enzyminhibitorwirkung werden 70 µl Pufferlösung, und 10 µl Enzymlösung mit 10 µl einer 10%igen (v/v) wäßrigen Dimethylsulfoxid-Lösung, die gegebenenfalls den Enzyminhibitor enthält für 15 Minuten inkubiert. Nach Zugabe von 10 µl einer 10%igen (v/v) wäßrigen Dimethylsulfoxidlösung, die 1 mmol/l des Substrates enthält, wird die Enzymreaktion fluoreszenzspektroskopisch verfolgt (328 nm (ex)/393 nm(em)). Die Enzymaktivität wird dargestellt als Extinktionszunahme/Minute. Die in Tab. 3 aufgeführten IC50-Werte wurden ermittelt als diejenige Inhibitorkonzentration die zu einer 50%igen Inhibierung des Enzyms führen. Die Pufferlösung enthält 0,05 Brij (Sigma, Deisenhofen, Deutschland) sowie 0,1 mol/l Tris/HCl, 0,1 mol/l NaCl, 0,01 mol/l CaCl₂ (pH = 7,5) für die Bestimmung der Hydroxamsäuren bis einschließlich Beispiel 33, bzw. für die Bestimmung der Carbonsäuren ab Beispiel 34 0,1 mo/l Piperazin-N,N'-bis[2-äthansulfonsäure] pH= 6,5.
Die Enzymlösung enthält 5 µg/ml einer der nach Ye et al. dargestellten Enzymdomänen. Die Substratlösung enthält 1 mmol/I desfluorogenen Substrates (7-Methoxycoumarin-4-yl)acetyl-Pro-Leu-Gly-Leu-3-(2',4'-dinitrophenyl)-L-2,3-diaminopropionyl-Ala-Arg-NH₂ (Bachem, Heidelberg, Deutschland).

**Tabelle 3**

| Beispiel Nr. | Stromelysin IC 50 [M] | Neutrophilen-Kollagenase IC 50 [M] |
|---|---|---|
| 1 | 3*10⁻⁷ | 2*10⁻⁸ |
| 2 | 2*10⁻⁸ | 2*10⁻⁹ |
| 3 | 3*10⁻⁸ | 2*10⁻⁹ |
| 4 | 7*10⁻⁷ | 1*10⁻⁷ |
| 5 | 6*10⁻⁶ | 3*10⁻⁷ |
| 6 | 5*10⁻⁷ | 3*10⁻⁸ |
| 8 | 3*10⁻⁶ | 2*10⁻⁷ |
| 9 | 4*10⁻⁷ | 8*10⁻⁷ |
| 10 | 3*10⁻⁷ | 1*10⁻⁷ |
| 11 | 4*10⁻⁷ | 7*10⁻⁸ |
| 12 | 4*10⁻⁷ | 2*10⁻⁷ |
| 13c | 2*10⁻⁸ | 2*10⁻⁹ |
| 14 | 3*10⁻⁸ | 2*10⁻⁹ |
| 15 | 1*10⁻⁷ | 1*10⁻⁸ |
| 17 | 1*10⁻⁷ | 2*10⁻⁸ |
| 18 | 3*10⁻⁸ | 3*10⁻⁹ |
| 19 | 2*10⁻⁶ | 3*10⁻⁷ |
| 20 | 1*10⁻⁸ | 1*10⁻⁹ |
| 21 | 2*10⁻⁸ | 2*10⁻⁹ |
| 22 | 3*10⁻⁸ | 8*10⁻⁹ |
| 23 | 8*10⁻⁸ | 8*10⁻⁹ |
| 24 | 6*10⁻⁸ | 2*10⁻⁸ |
| 25 | 4*10⁻⁷ | 3*10⁻⁷ |
| 26 | 6*10⁻⁶ | 3*10⁻⁷ |
| 27 | 3*10⁻⁸ | 4*10⁻⁹ |
| 28 | 2*10⁻⁶ | 7*10⁻⁷ |
| 29 | 2*10⁻⁸ | 4*10⁻⁹ |
| 31 | 2*10⁻⁸ | 3*10⁻⁹ |
| 32 | 6*10⁻⁸ | 7*10⁻⁹ |
| 33 | 3*10⁻⁷ | 7*10⁻⁸ |
| 34 | 5*10⁻⁷ | 1*10⁻⁸ |
| 35 | 1*10⁻⁷ | 5*10⁻⁹ |
| 36 | | 3*10⁻⁶ |
| 39 | 1*10⁻⁷ | 1*10⁻⁹ |
| 41(13b) | 2*10⁻⁷ | 9*10⁻⁹ |
| 42 | 5*10⁻⁷ | 2*10⁻⁸ |
| 43 | 2*10⁻⁶ | 2*10⁻⁷ |
| 44 | 2*10⁻⁷ | 3*10⁻⁸ |
| 45 | 3*10⁻⁶ | 3*10⁻⁷ |
| 46 | 3*10⁻⁶ | 3*10⁻⁷ |
| 50 | 6*10⁻⁷ | 3*10⁻⁸ |
| 51 | 5*10⁻⁷ | 2*10⁻⁸ |
| 52 | 1*10⁻⁶ | 4*10⁻⁸ |
| 53 | 5*10⁻⁷ | 2*10⁻⁸ |
| 57 | 2*10⁻⁶ | 1*10⁻⁷ |

### 2. Proteoglycan-Degradations-Assay

### Prinzip des Assays:

In dem Proteoglykan-Degradationsassay wird das Ausmaß der Spaltung von nativem, bovinen Aggrecan, dem wichtigsten Proteoglykan des Gelenkknorpel, gemessen. Der Nachweis der freigesetzten Proteoglykanfragmente erfolgt mit dem monoklonalen Antikörper 5-D-4, der die Keratansulfatseitenketten erkennt, die carboxyterminal an der G2 Domäne des Aggrecans liegen. Damit erfaßt der Assay in erster Linie die pathologisch bedeutsamen Spaltungen, die in der Interglobulären Domäne des Aggrecans stattfinden.

Nach Zugabe an Verbindungen der Formel 1 und des Enzyms in Form der katalytischen Domäne des Stromelysin-1, wird die, nach der Spaltung verbliebene Menge an Hyaluronsäure-gebundenem Aggrecan gemessen. Je mehr Aggrecan dabei detektiert wird, um so niedriger ist die Restaktivität des Enzyms. Die Konzentrationen an Verbindungen der Formel I, bei der die eingesetzte Enzymaktivität (=100% Restaktivität) um die Hälfte (= 50% Restaktivität) reduziert wird, werden durch die IC50-Werte in Tabelle 3 dargestellt.

### Beschreibung der Versuchsdurchführung:

Die Näpfchen von 96well-Mikrotiterplatten (Nunc, Maxisorp) werden mit jeweils 100 µl Hyaluronsäurelösung (25 µg/ml Hyaluronsäure (Sigma) in PBS) für 12 h bei Raumtemperatur (RT) inkubiert. Nach Absaugen der Hyaluronsäurelösung werden die noch freien Proteinbindungsstellen der Näpfchen mit je 100 ml einer 5%igen Lösung von Bovinem Serumalbumin (BSA), 0,05% Tween20 in PBS für 1 h bei RT abgesättigt. Danach werden die Näpfchen mit Proteoglykan beschichtet, indem die Näpfchen mit je 100 µl einer Lösung von Bovinem Nasenproteoglycan (ICI) (200 µg/ml in 1 x PBS, 5 mg/ml BSA, 0,05% Tween20) für 1 h bei RT inkubiert werden. Zweimaliges Waschen mit 1 x PBS, 0,1 % Tween20 entfernt die ungebundenen Proteoglykane. Anschließend werden für den eigentlichen Assay 60 ng gereinigter katalytischer Domäne von Stromelysin-1 (Rekombinante Expression und Reinigung siehe Ye et al. (1992) plus entsprechende Konzentrationen an zu testendem Inhibitor in 100 µl Verdaupuffer (100 mM MES pH 6,0, 100 mM NaCl, 10 mM CaCl₂, 0,05% Brij) in die Näpfchen pipettiert und für 3 h bei RT inkubiert. Nach zweimaligem Waschen mit 1x PBS, 0,1% Tween20 werden die Näpfchen für 1 h bei RT mit 100 µl Lösung des Detektionsantikörpers (Monoklonaler Antikörper Klon 5-D-4 (ICI), immunoreaktiv mit den Keratansulfatseitenketten des Proteoglykans, in der Verdünnung 1:1000 in 1x PBS, 5 mg/ml BSA, 0,05% Tween20) inkubiert. Nach zweimaligem Waschen mit 1x PBS, 0,1% Tween20 erfolgt die Immunoreaktion der gebundenen Detektionsantikörper mit 100 µl pro Näpfchen an Nachweisantikörperlösung (Ziege anti Maus IgG, markiert mit Peroxidase (Dianova), verdünnt 1:1000 in 1x PBS, 5 mg/ml BSA, 0,05 % Tween20) für 1 h bei RT. Nach erneutem zweimaligen Waschen (wie oben) wird die Farbreaktion mit je 100 µl 2mg/ml ABTS, aktiviert mit H₂O₂ eingeleitet. Die Messung der Reaktionsprodukte erfolgt im ELISA-Reader bei 405 mm Lichtwellenlänge. Die Tabelle 4 zeigt die Ergebnisse.

**Tabelle 4**

| Beispiel-Nr. | Proteoglykan-Degradation IC50 [M] |
|---|---|
| 2 | 8,5*10⁻⁸ |
| 9 | 1,6*10⁻⁶ |
| 13c | 5,1*10⁻⁸ |
| 14 | 6,7*10⁻⁹ |
| 18 | 4,1*10⁻⁸ |
| 20 | 1,3*10⁻⁷ |
| 21 | 6,5*10⁻⁸ |
| 29 | 2,5*10⁻⁸ |

## Patentansprüche

1. Verbindung der Formel I 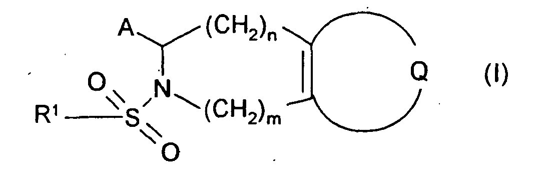 und/oder eine gegebenenfalls stereoisomere Form der Verbindung der Formel I und/oder ein physiologisch verträgliches Salz der Verbindung der Formel I, wobei für den Fall i)
R¹ für
a) einen Rest der Formel II 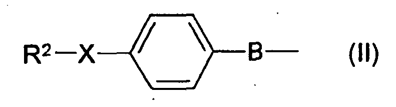
b) einen Rest der Formel III 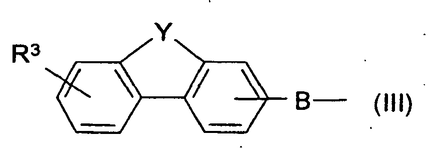
c) einen Rest der Formel IV 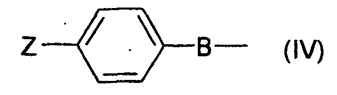 worin Z ein Rest eines Heterocyclus oder substituierten Hetereocyclus ist, wie
1) Pyrrol,
2) Thiazol,
3) Pyrazol,
4) Pyridin,
5) Imidazol,
6) Pyrrolidin,
7) Piperidin,
8) Thiophen,
9) Oxazol,
10) Isoxazol,
11) Morpholin oder
12) Piperazin, steht, oder
d) einen Rest der Formel V 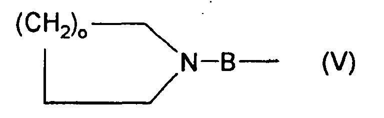 worin o die Zahl 1 oder 2 ist, und eines der Kohlenstoffatome im Ring gegebenenfalls durch -O- oder -S- ersetzt ist, bedeutet, und
Q als Teil der Strukturformel I 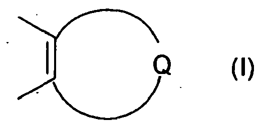
1) für den Strukturteil VI 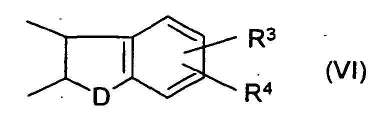
2) den Strukturteil VII 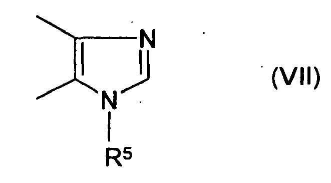
3) für den Strukturteil VIII 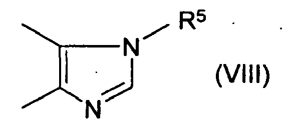
4) den Strukturteil IX 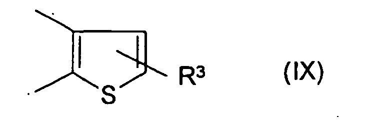 oder
5) den Strukturteil X 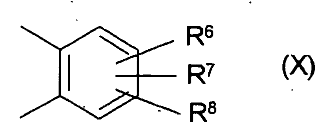 steht, worin D NR⁴ oder S bedeutet,
R² für
1) Phenyl oder
2) Phenyl ein- bis dreifach substituiert durch
2.1. Hydroxy,
2.2. -O-R¹⁰, worin R¹⁰
1) (C₁-C₆)-Alkyl,
2) (C₃-C₆)-Cycloalkyl,
3) Benzyl oder
4) Phenyl bedeutet,
2.3. -COOH,
2.4. (C₁-C₆)-Alkyl,
2.5. (C₃-C₆)-Cycloalkyl-O-(C₁-C₄)-alkyl,
2.6. Halogen,
2.7. -CN,
2.8. -NO₂,
2.9. -CF₃,
2.10. -O-C(O)-R¹⁰ und R¹⁰ wie oben definiert ist,
2.11. -O-C(O)-Phenyl, ein- oder zweifach substituiert durch R³,
2.12. -C(O)-O-R¹⁰ und R¹⁰ wie oben definiert ist,
2.13. Methylendioxo,
2.14. -C(O)-NR¹¹R¹², worin
R¹¹ und R¹² gleich oder verschieden sein können und
1) Wasserstoffatom,
2) (C₁-C₄)-Alkyl oder
3) Benzyl bedeuten oder
4) R¹¹ und R¹² zusammen mit dem N-Atom an das sie gebunden sind einen Pyrrolidin-, Piperidin-, Morpholin- oder Piperazinrest bilden, oder
2.15. -NR¹³R¹⁴, worin R¹³
Wasserstoffatom oder (C₁-C₄)-Alkyl bedeutet und
R¹⁴
1) Wasserstoffatom,
2) (C₁-C₄)-Alkyl,
3) Benzyl,
4) -C(O)-R¹⁰ oder
5) -C(O)-O-R¹⁰ bedeutet und R¹⁰ wie oben definiert ist,
R³ und R⁴ gleich oder verschieden sind und
1) Wasserstoffatom,
2) (C₁-C₅)-Alkyl,
3) (C₁-C₅)-Alkoxy,
4) Halogen,
5) Hydroxy,
6) -O-C(O)-R¹⁰ und R¹⁰ wie oben definiert ist, bedeuten, oder
7) R³ und R⁴ zusammen den Rest -O-CH₂-O- bilden,
R⁵ für
a) Wasserstoffatom,
b) (C₁-C₅)-Alkyl oder
c) Benzyl steht, und
R⁶, R⁷ und R⁸ gleich oder verschieden sind und für
a) Wasserstoffatom stehen, oder
b) die Bedeutung für den Fall i) von R² unter den Punkten 2.1 bis 2.14, haben, und
n für Null, oder 2 steht,
m für Null,1 oder 2 steht, wobei die Summe von n und m 1, 2 oder 3 ist,
mit Ausnahme des Falles, dass
A für HO-C(O)- steht, Q als Teil der Strukturformel I für den Strukturteil X steht, R⁶, R⁷ und R⁸ für Wasserstoffatom oder -O-Methyl stehen, n für 1 steht, m für 1 steht und R¹ für den Rest der Formel V steht, worin o die Zahl 2 ist und eines der Kohlenstoffatome durch -0- ersetzt ist, oder
wobei für den Fall ii)
R¹ für
1) Phenyl oder
2) Phenyl, ein- bis dreifach substituiert durch R², wobei R² wie für den Fall i) unter den Punkten 2.1 bis 2.15 definiert ist,
Q für den Strukturteil X steht und
R⁶, R⁷ und R⁸ gleich oder verschieden sind und wie oben definiert sind,
n 1 bedeutet und
m 1 bedeutet,
mit Ausnahme der Fälle, dass
A für HO-C(O)- steht, R⁶, R⁷ und R⁸ für Wasserstoffatom stehen, n für 1 steht, m für 1 steht und R¹ für Phenyl steht, das einfach durch Methyl, Nitro oder Amino substituiert ist oder A für HO-C(O)- steht, R⁶, R⁷ und R⁸ für Wasserstoffatom stehen, n für 1 steht, m für 1 steht und R¹ für Phenyl steht, das zweifach durch Amino und Chlor substituiert ist oder
wobei für den Fall iii)
R¹, Q, R⁶, R⁷ und R⁸ gleich oder verschieden sind und die für den Fall ii) genannte Bedeutung haben, m und n Null, 1 oder 2 bedeuten und wobei die Bedeutung von n und m nicht gleich ist,
mit Ausnahme des Falles, dass
A für HO-C(O)- steht, R⁶, R⁷ und R⁸ für Wasserstoffatom stehen, n für 1 steht, m für Null steht und R¹ für Phenyl steht, das einfach durch Methyl substituiert ist
und X für
a) eine kovalente Bindung,
b) -O-,
c) -S-,
d) -S(O)-,
e) -S(O)₂-,
f) -C(O)- oder
g) -C(OH)- steht, und
Y für
a) -O- oder
b) -S- steht, und
A für HO-NH-C(O)- oder HO-C(O)- steht und
B für a) -(CH₂)q-, worin q Null, 1, 2, 3 oder 4 bedeutet, oder b) -CH=CH- steht.

2. Verbindung der Formel I gemäß Anspruch 1 und/oder ein physiologisch verträgliches Salz der Verbindung der Formel I und/oder eine gegebenenfalls stereosiomere Form der Verbindung der Formel I, wobei
R¹ für den Fall i) für einen Rest der Formel II oder III steht und Q für den Strukturteil VI, VII, VIII oder X steht,
R¹ für den Fall ii) für Phenyl oder Phenyl, ein- bis dreifach substituiert durch Methoxy, steht und Q für den Strukturteil X steht, oder
R¹ für den Fall iii) für Phenyl, Q für den Strukturteil X steht, n Null und m 2 bedeutet, und
A für HO-NH-C(O)- oder HO-C(O)- steht,
B für eine kovalente Bindung steht,
X für Sauerstoffatom oder eine kovalente Bindung steht, und
R² für Phenyl oder Phenyl substituiert durch
a) Hydroxy,
b) -O-R¹⁰, worin R¹⁰ (C₁-C₃)-Alkyl oder Benzyl bedeutet,
c) (C₁-C₂)-Alkyl,
d) Fluor oder Chlor,
e) -CN,
f) -CF₃ oder
g) -NR¹³R¹⁴, worin R¹³ und R¹⁴ (C₁-C₃)-Alkyl bedeuten
R³, R⁴, R⁵, R⁶, R⁷ und R⁸ gleich oder verschieden sind und für
a) Wasserstoffatom,
b) Methoxy,
c) Methylendioxo,
d) Amino oder
e) Hydroxy stehen.

3. Verbindung der Formel gemäß der Ansprüche 1 oder 2, wobei man
R-2-(Biphenylsulfonyl)-1,2,3,4-tetrahydroisochinolin-3-hydroxamsäure,
R-2-(4-Chlor-biphenylsulfonyl)-1,2,3,4-tetrahydroisochinolin-3-hydroxamsäure,
R-2-(4-Chlor-biphenylsulfonyl)-1,2,3,4-tetrahydroisochinolin-3-carbonsäure,
R-2-(4-Phenoxybenzolsulfonyl)-1,2,3,4-tetrahydroisochinolin-3-hydroxymsäure,
R-2-(4-Phenoxybenzolsulfonyl)-1,2,3,4-tetrahydroisochinolin-3-carbonsäure,
R-2-(4-(4-Dimethylaminophenoxy)-benzolsulfonyl)-1,2,3,4-tetrahydroisochinolin-3-hydroxamsäure,
R-2-(4-Dimethylaminobiphenylsulfonyl)-1,2,3,4-tetrahydroisochinolin-3-carbonsäure,
R-2-(4-Benzoylphenylsülfonyl)-1,2,3,4-tetrahydroisochinolin-3-hydroxamsäure,
R-2-(4-Methoxybenzolsulfonyl)-7-hydroxy-1,2,3,4-tetrahydroisochinolin-3-hydroxamsäure,
R-2-(4-Methoxybenzolsulfonyl)-7-nitro-1,2,3,4-tetrahydroisochinolin-3-hydroxamsäure,
2-(4-Methoxybenzolsulfonyl)-6,7-propylen-1,2,3,4-tetrahydroisochinolin-1-hydroxamsäure,
R-5-(4-Methoxybenzolsulfonyl)-4,5,6,7-tetrahydro-1H-imidazo-(4,5-c)-pyridin-6-hyd roxa msä u re,
R-2-(4-Methoxybenzolsulfonyl)-1,2,3,4-tetrahydro-9H-pyrido-(3,4-c)-indol-3-hydroxamsäure,
R-2-(4-Phenoxybenzolsulfonyl)-1,2,3,4-tetrahydro-9H-pyrido-(3,4-c)-indol-3-hydroxamsäure einsetzt.

4. Verbindungen der Formel I gemäß einem oder mehreren der Ansprüche 1 bis 3, **dadurch gekennzeichnet, daß** das zentrale Kohlenstoffatom zwischen Amino- und Hydroxamsäuregruppe als R-Enantiomer vorliegt.

5. Verfahren zur Herstellung der Verbindung der Formel I gemäß einem oder mehreren der Ansprüche 1 bis 4, **dadurch gekennzeichnet, daß** man
a) eine Iminosäure der Formel XI 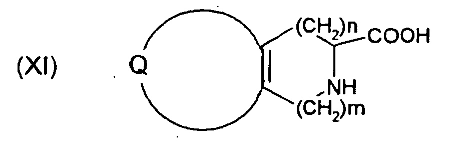 worin Reste Q, sowie n und m wie die Formel I definiert sind, mit einem
(C₁-C₄)-Alkohol oder einem Benzylalkohol zu der Verbindung der Formel XII umsetzt, 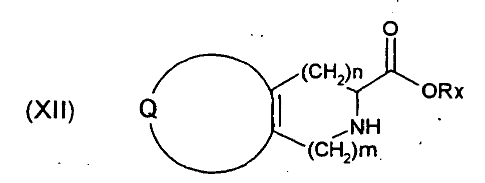 worin Rₓ (C₁-C₄)-Alkyl oder Benzyl bedeutet, oder
b) eine nach Verfahren a) hergestellte Verbindung der Formel XII mit der Verbindung der Formel XIII, 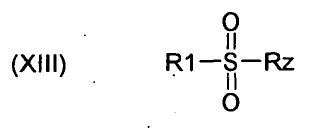 worin R¹ wie in Formel I definiert ist und R_{z} Chloratom, Imidazolyl oder-OH bedeutet, in Gegenwart einer Base zu einer Verbindung der Formel XIV umsetzt, 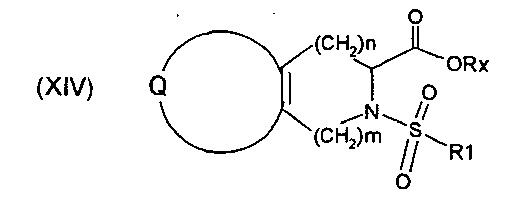 worin Q, R¹, n und m wie die Formel I definiert sind und Rₓ wie in Formel XII definiert ist, oder
c) eine nach Verfahren a) hergestellte Verbindung der Formel XII mit einer Base umsetzt und anschließend mit einer Verbindung der Formel XIII zu einer Verbindung der Formel XIV umsetzt, oder
d) eine Verbindung der Formel XI mit einer Verbindung der Formel XIII zu einer Verbindung der Formel XV umsetzt 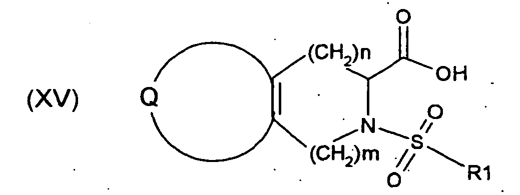 worin Q, R¹, n und m wie in Formel I definiert sind, oder
e) eine Verbindung der Formel XIV zu Verbindung XV umsetzt oder,
f) eine nach Verfahren b) oder c) hergestellte Verbindung der Formel XIV mit dem Hydroxylamin der Formel XVI,
(XVI) H₂N-ORy
worin Ry Wasserstoffatom oder eine Sauerstoffgruppe bedeutet, zu der Verbindung der Formel I umsetzt und gegebenenfalls die Sauerstoffschutzgruppe abspaltet, oder
g) eine nach Verfahren d) oder e) hergestellte Verbindung der Formel XV mit dem Hydroxylamin der Formel XVI zu der Verbindung der Formel I umsetzt, oder
h) eine nach den Verfahren f) oder g) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppe in die reinen Enantiomeren auftrennt, oder
i) die nach den Verfahren f), g), und h) hergestellte Verbindung der Formel I entweder in freier Form isoliert oder im Falle des Vorliegens von sauren oder basischen Gruppen gegebenenfalls in physiologisch verträgliche Salze umwandelt.

6. Arzneimittel, enthaltend eine wirksame Menge von mindestens einer Verbindung der Formel I 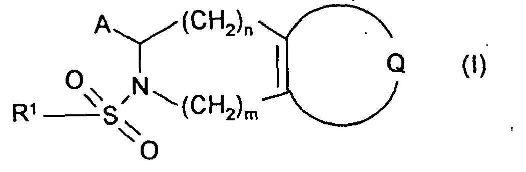 und/oder eine gegebenenfalls stereoisomere. Form der Verbindung der Formel I und/oder ein physiologisch verträgliches Salz der Verbindung der Formel I, wobei für den Fall i)
R¹ für
a) einen Rest der Formel II 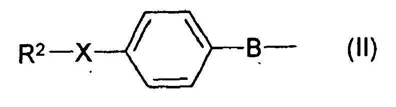
b) einen Rest der Formel III 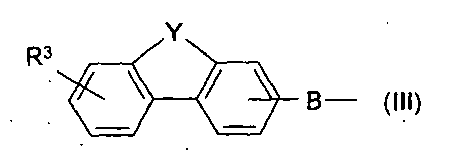
c) einen Rest der Formel IV 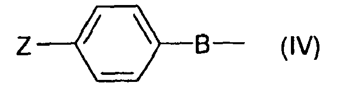 worin Z ein Rest eines Heterocyclus oder substituierten Hetereocyclus ist, wie
1) Pyrrol,
2) Thiazol,
3) Pyrazol,
4) Pyridin,
5) Imidazol,
6) Pyrrolidin,
7) Piperidin,
8) Thiophen,
9) Oxazol,
10) Isoxazol,
11) Morpholin oder
12) Piperazin, steht, oder
d) einen Rest der Formel V 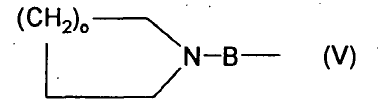 worin o die Zahl 1 oder 2 ist, und eines der Kohlenstoffatome im Ring gegebenenfalls durch -O- oder -S- ersetzt ist, bedeutet, und
Q als Teil der Strukturformel I 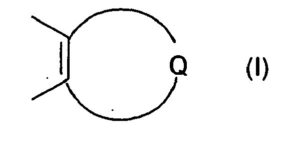
1) für den Strukturteil VI 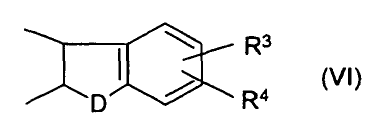
2) den Strukturteil Vil 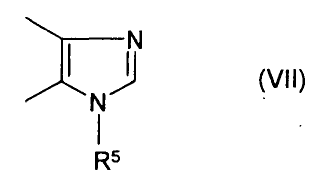
3) für den Strukturteil VIII 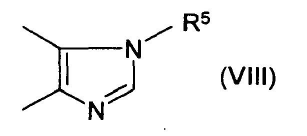
4) den Strukturteil IX 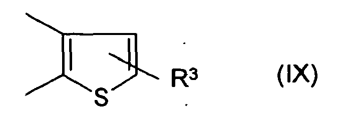 oder
5) den Strukturteil X 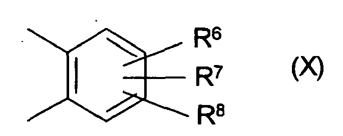 steht, worin D NR⁴ oder S bedeutet,
R² für
1) Phenyl oder
2) Phenyl ein- bis dreifach substituiert durch
2.1. Hydroxy,
2.2. -O-R¹⁰, worin R¹⁰
1)(C₁-C₆)-Alkyl,
2) (C₃-C₆)-Cycloalkyl,
3) Benzyl oder
4) Phenyl bedeutet,
2.3. -COOH,
2.4. (C₁-C₆)-Alkyl,
2.5. (C₃-C₆)-Cycloalkyl-O-(C₁-C₄)-alkyl,
2.6. Halogen,
2.7. -CN,
2.8. -NO₂,
2.9. -CF₃,
2.10. -O-C(O)-R¹⁰ und R¹⁰ wie oben definiert ist,
2.11. -O-C(O)-Phenyl, ein- oder zweifach substituiert durch R³,
2.12. -C(O)-O-R¹⁰ und R¹⁰ wie oben definiert ist,
2.13. Methylendioxo,
2.14. -C(O)-NR¹¹R¹², worin
R¹¹ und R¹² gleich oder verschieden sein können und
1) Wasserstoffatom,
2) (C₁-C₄)-Alkyl oder
3) Benzyl bedeuten oder
4) R¹¹ und R¹² zusammen mit dem N-Atom an das sie gebunden sind einen Pyrrolidin-, Piperidin-, Morpholin- oder Piperazinrest bilden, oder
2.15. -NR¹³R¹⁴, worin R¹³
Wasserstoffatom oder (C₁-C₄)-Alkyl bedeutet und
R¹⁴
1) Wasserstoffatom,
2) (C₁-C₄)-Alkyl,
3) Benzyl,
4) -C(O)-R¹⁰ oder
5) -C(O)-O-R¹⁰ bedeutet und R¹⁰ wie oben definiert ist,
R³ und R⁴ gleich oder verschieden sind und
1) Wasserstoffatom,
2) (C₁-C₅)-Alkyl,
3) (C₁-C₅)-Alkoxy,
4) Halogen,
5) Hydroxy,
6) -O-C(O)-R¹⁰ und R¹⁰ wie oben definiert ist, bedeuten, oder
7) R³ und R⁴ zusammen den Rest -O-CH₂-O- bilden,
R⁵ für
a) Wasserstoffatom,
b) (C₁-C₅)-Alkyl oder
c) Benzyl steht, und
R⁶, R⁷ und R⁸ gleich oder verschieden sind und für
a) Wasserstoffatom stehen, oder
b) die Bedeutung für den Fall i) von R² unter den Punkten 2.1 bis 2.14, haben, und
n für Null, 1 oder 2 steht,
m für Null, 1 oder 2 steht, wobei die Summe von n und m 1, 2 oder 3 ist, oder
wobei für den Fall ii)
R¹ für
1) Phenyl oder
2) Phenyl, ein- bis dreifach substituiert durch R², wobei R² wie für den Fall i) unter den Punkten 2.1 bis 2.15 definiert ist,
Q für den Strukturteil X steht und
R⁶, R⁷ und R⁸ gleich oder verschieden sind und wie oben definiert sind,
n 1 bedeutet und
m 1 bedeutet, oder
wobei für den Fall iii)
R¹, Q, R⁶, R⁷ und R⁸ gleich oder verschieden sind und die für den Fall ii) genannte Bedeutung haben, m und n Null, 1 oder 2 bedeuten und wobei die Bedeutung von n und m nicht gleich ist, und
X für
a) eine kovalente Bindung,
b) -O-,
c) -S-,
d) -S(O)-,
e) -S(O)₂-,
f) -C(O)- oder
g) -C(OH)- steht, und
Y für
a) -O- oder
b) -S- steht, und
A für HO-NH-C(O)- oder HO-C(O)- steht und
B für a) -(CH₂)_{q}-, worin q Null,1, 2, 3 oder 4 bedeutet, oder b) -CH=CH- steht,
neben physiologisch annehmbaren Hilfs- und Trägerstoffen, gegebenenfalls weiteren Zusatzstoffen und/oder anderen Wirkstoffen.

7. Arzneimittel gemäß Anspruch 6, enthaltend eine wirksame Menge von mindestens einer Verbindung der Formel 1, wobei
R¹ für den Fall i) für einen Rest der Formel 11 oder III steht und Q für den Strukturteil VI, VII, VIII oder X steht,
R¹ für den Fall ii) für Phenyl oder Phenyl, ein- bis dreifach substituiert durch Methoxy, steht und Q für den Strukturteil X steht, oder
R¹ für den Fall iii) für Phenyl, Q für den Strukturteil X steht, n Null und m 2 bedeutet, und
A für HO-NH-C(O)- oder HO-C(O)- steht,
B für eine kovalente Bindung steht,
X für Sauerstoffatom oder eine kovalente Bindung steht, und
R² für Phenyl oder Phenyl substituiert durch
a) Hydroxy,
b) -O-R¹⁰, worin R¹⁰ (C₁-C₃)-Alkyl oder Benzyl bedeutet,
c) (C₁-C₂)-Alkyl,
d) Fluor oder Chlor,
e) -CN,
f) -CF₃ oder
g) -NR¹³R¹⁴, worin R¹³ und R¹⁴ (C₁-C₃)-Alkyl bedeuten
R³, R⁴, R⁵, R⁶, R⁷ und R⁸ gleich oder verschieden sind und für
a) Wasserstoffatom,
b) Methoxy,
c) Methylendioxo,
d) Amino oder
e) Hydroxy stehen.

8. Arzneimittel gemäß der Ansprüche 6 oder 7, enthaltend eine wirksame Menge von mindestens einer Verbindung der Formel I, wobei man
R-2-(Biphenylsulfonyl)-1,2,3,4-tetrahydroisochinolin-3-hydroxamsäure,
R-2-(4-Chlor-biphenylsülfonyl)-1,2,3,4-tetrahydroisochinolin-3-hydroxamsäure,
R-2-(4-Chlor-biphenylsulfonyl)-1,2,3,4-tetrahydroisochinolin-3-carbonsäure,
R-2-(4-Phenoxybenzolsulfonyl)-1,2,3,4-tetrahydroisochinolin-3-hydroxymsäure,
R-2-(4-Phenoxybenzolsulfonyl)-1,2,3,4-tetrahydroisochinolin-3- carbonsäure,
R-2-(4-(4-Dimethylaminophenoxy)-benzolsulfonyl)-1,2,3,4-tetrahydroisochinolin-3-hydroxamsäure,
R-2-(4-Dimethylaminobiphenylsulfonyl)-1,2,3,4-tetrahydroisochinolin-3-rarbonsäure,
R-2-(4-Benzoylphenylsulfonyl)-1,2,3,4-tetrahydroisochinolin-3-hydroxamsäure,
R-2-(4-Methoxybenzolsulfonyl)-7-hydroxy-1,2,3,4-tetrahydroisochinolin-3-hydroxamsäure,
R-2-(4-Methoxybenzolsulfonyl)-7-nitro-1,2,3,4-tetrahydroisochinolin-3-hydroxamsäure,
2-(4-Methoxybenzolsulfonyl)-6,7-propylen-1,2,3,4-tetrahydroisochinolin-1-hydroxamsäure,
R-5-(4-Methoxybenzolsulfonyl)-4,5,6,7-tetrahydro-1H-imidazo-(4,5-c)-pyridin-6-hydroxamsäure,
R-2-(4-Methoxybenzolsulfonyl)-1,2,3,4-tetrahydro-9H-pyrido-(3,4-c)-indol-3-hydroxamsäure oder
R-2-(4-Phenoxybenzolsulfonyl)-1,2,3,4-tetrahydro-9H-pyrido-(3,4-c)-indol-3-hydroxamsäure einsetzt.

9. Arzneimittel gemäß einem oder mehreren der Ansprüche 6 bis 8, enthaltend eine wirksame Menge von mindestens einer Verbindung der Formel 1, wobei das zentrale Kohlenstoffatom zwischen Amino- und Hydroxamsäuregruppe als R-Enantiomer vorliegt.

10. Verwendung von mindestens einer Verbindung der Formel 1 gemäß einem oder mehreren der Ansprüche 6 bis 9, zur Herstellung von Arzneimitteln zur Prophylaxe und Therapie von Erkrankungen, an deren Verlauf eine verstärkte Aktivität von Matrix-abbauenden Metallproteinasen beteiligt ist.

11. Verwendung nach Anspruch 10, fürdie Behandlungvon Erkrankungen des Bindegewebes wie Kollagenosen, Periodontalerkrankungen, Wundheilungsstörungen oder chronische Erkrankungen des Bewegungsapparates wie entzündlich, immunologisch oder stoffwechselbedingte akute und chronische Arthritiden, Arthropathien, Myalgien und Störungen des Knochenstoffwechsels oder degenerative Gelenkserkrankungen wie Osteoarthrosen, Spondylosen, Knorpelschwund nach Gelenktrauma oder längerer Gelenksruhigstellung nach Meniskus- oder Patellaverletzungen oder Bänderrissen oder Behandlung der Ulceration, Artherosklerose und Stenosen oder Hemmung der Tumor Nekrose Faktor Freisetzung oder Behandlung von Entzündungen, Krebserkrankungen, Tumormetastasenbildung, Kachexie, Anorexie und septischen Schock.

12. Verfahren zur Herstellung eines Arzneimittels gemäß der Ansprüche 6 bis 9, **dadurch gekennzeichnet, dass** man mindestens eine Verbindung der Formel gemäß einem oder mehreren der Ansprüche 6 bis 9 und/oder mindestens ein physiologisch verträgliches Salz der Verbindung der Formel und/oder eine gegebenenfalls stereoisomere Form der Verbindung der Formel I mit physiologisch annehmbaren Hilfs- und Trägerstoffen und gegebenenfalls weiteren Zusatzstoffen und/oder anderen Wirkstoffen in eine geeignete Darreichungsform bringt.

## Claims

1. A compound of the formula I 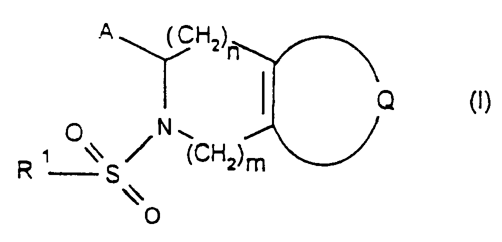 and/or an optionally stereoisomeric form of the compound of the formula I and/or a physiologically tolerable salt of the compound of the formula I, where in the case i)
R¹ is
a) a radical of the formula II 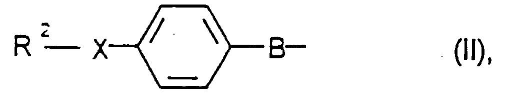
b) a radical of the formula III 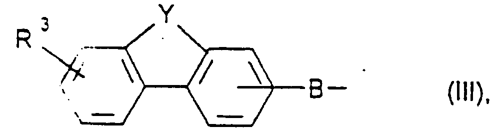
c) a radical of the formula IV 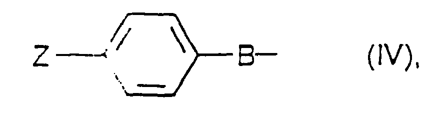 where Z is a radical of a heterocycle or a substituted heterocycle such as
1) pyrrole,
2) thiazole,
3) pyrazole,
4) pyridine,
5) imidazole,
6) pyrrolidine,
7) piperidine,
8) thiophene,
9) oxazole,
10) isoxazole,
11) morpholine or
12) piperazine, or
d) a radical of the formula V 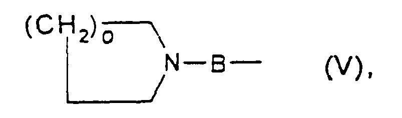 where o is the number 1 or 2 and one of the carbon atoms in the ring may be replaced by -O- or -S-, and
Q as part of the structural formula I 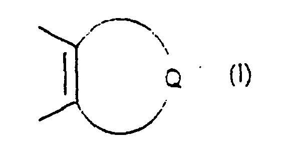
1) is the structural moiety VI 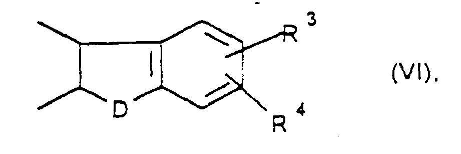
2) the structural moiety VII 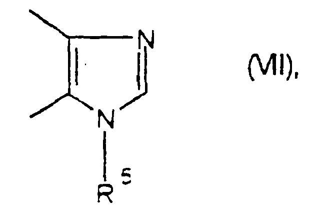
3) is the structural moiety VIII 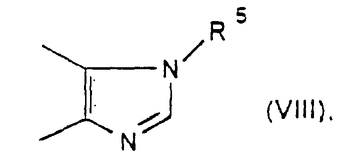
4) the structural moiety IX 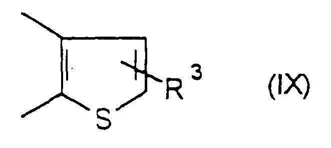 or
5) is the structural moiety X 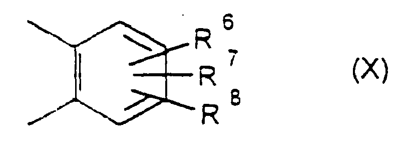
where D is NR⁴ or S,
R² is
1) phenyl or
2) phenyl which is mono- to trisubstituted by
2.1 hydroxyl,
2.2 -O-R¹⁰, where R¹⁰
1) is (C₁-C₆) -alkyl,
1) is (C₁-C₆)-alkyl,
2) is (C₃-C₆)-cycloalkyl,
3) is benzyl or
4) is phenyl,
2.3 -COOH,
2.4 (C₁-C₆)-alkyl,
2.5 (C₃-C₆)-cycloalkyl-O-(C₁-C₄)-alkyl,
2.6 halogen,
2.7 -CN,
2 . 8 -NO₂,
2.9 -CF₃,
2.10 -O-C(O)-R¹⁰ and R¹⁰ is as defined above,
2.11 -O-C(O)-phenyl, mono- or disubstituted by R³.
2.12 -C(O)-O-R¹⁰ and R¹⁰ is as defined above,
2.13 methylenedioxo,
2.14 -C(O)-NR¹¹R¹², where
R¹¹ and R¹² may be identical or different and each is
1) a hydrogen atom,
2) (C₁-C₄) -alkyl or
3) benzyl or
4) R¹¹ and R¹² together with the linking nitrogen atom form a pyrrolidine, piperidine, morpholine or piperazine radical, or
2.15 -NR¹³R¹⁴, where R¹³ is a hydrogen atom or (C₁-C₄)-alkyl and
R¹⁴
1) is a hydrogen atom,
2) is (C₁-C₄)-alkyl,
3) is benzyl,
4) is -C(O)-R¹⁰ or
5) is -C(O)-O-R¹⁰ and R¹⁰ is as defined above,
R³ and R⁴ are identical or different and each is
1) a hydrogen atom,
2) (C₁-C₅)-alkyl,
3) (C₁-C₅)-alkoxy,
4) halogen,
5) hydroxyl,
6) -O-C (O) -R¹⁰ and R¹⁰ is as defined above, or
7) R³ and R⁴ together form the radical -O-CH₂-O-,
R⁵ is
a) a hydrogen atom,
b) (C₁-C₅)-alkyl or
c) benzyl, and
R⁶, R⁷ and R⁸ are identical or different and each is
a) a hydrogen atom, or
b) has, in the case of i), the meaning of R² under items 2.1 to 2.14, and
n is zero, 1 or 2,
m is zero, 1 or 2, the sum of n and m being 1, 2 or 3,
except for the case where
A is HO-C(O)-, Q as part of the structural formula I is the structural moiety X, R⁶, R⁷ and R⁸ are hydrogen atoms or -O-methyl, n is 1, m is 1 and R¹ is the radical of the formula V where o is the number 2 and one of the carbon atoms is replaced by -O-, or
where in the case ii)
R¹ is
1) phenyl or
2) phenyl, mono- to trisubstituted by R² where R² is as defined for the case i) under items 2.1 to 2.15,
Q is the structural moiety X and
R⁶, R⁷ and R⁸ are identical or different and each is defined as above,
n is 1 and
m is 1,
except for the cases where
A is HO-C(O)-, R⁶, R⁷ and R⁸ are hydrogen atoms, n is 1, m is 1 and R¹ is phenyl which is monosubstituted by methyl, nitro or amino or
A is HO-C(O)-, R⁶, R⁷ and R⁸ are hydrogen atoms, n is 1, m is 1 and R¹ is phenyl which is disubstituted by amino and chlorine or
where in the case iii)
R¹, Q, R⁶, R⁷ and R⁸ are identical or different and each has the meaning mentioned for the case ii), m and n are zero, 1 or 2 and where the meanings of n and m are not identical,
except for the case where
A is HO-C(O)-, R⁶, R⁷ and R⁸ are hydrogen atoms, n is 1, m is zero and R¹ is phenyl which is monosubstituted by methyl
and X is
a) a covalent bond,
b) -O-,
c) -S-,
d) -S(O)-,
e) -S(O)₂-,
f) -C(O)- or
g) -C(OH)-, and
Y is
a) -O- or
b) -S-, and
A is HO-NH-C(O)- or HO-C(O)- and
B is a) -(CH₂)_{q}-, where q is zero, 1, 2, 3 or 4, or b) is -CH=CH-.

2. The compound of the formula I as claimed in claim 1 and/or a physiologically tolerable salt of the compound of the formula I and/or an optionally stereoisomeric form of the compound of the formula I, where
R¹ in the case i) is a radical of the formula II or III and Q is the structural moiety VI, VII, VIII or X,
R¹ in the case ii) is phenyl or phenyl, mono- to trisubstituted by methoxy, and Q is the structural moiety X, or
R¹ in the case iii) is phenyl, Q is the structural moiety X, n is zero and m is 2, and
A is HO-NH-C(O)- or HO-C(O)-,
B is a covalent bond,
X is an oxygen atom or a covalent bond, and
R² is phenyl or phenyl substituted by
a) hydroxyl,
b) -O-R¹⁰, where R¹⁰ is (C₁-C₃) -alkyl or benzyl,
c) (C₁-C₂)-alkyl,
d) fluorine or chlorine,
e) -CN,
f) -CF₃ or
g) -NR¹³R¹⁴, where R¹³ and R¹⁴ are each
(C₁-C₃)-alkyl,
R³, R⁴, R⁵, R⁶, R⁷ and R⁸ are identical or different and each is
a) a hydrogen atom,
b) methoxy,
c) methylenedioxo,
d) amino or
e) hydroxyl.

3. The compound of the formula I as claimed in claim 1 or 2, wherein
R-2-(biphenylsulfonyl)-1,2,3,4-tetrahydroisoquinoline-3-hydroxamic acid,
R-2-(4-chlorobiphenylsulfonyl)-1,2,3,4-tetrahydroisoquinoline-3-hydroxamic acid,
R-2-(4-chlorobiphenylsulfonyl)-1,2,3,4-tetrahydroisoquinoline-3-carboxylic acid,
R-2-(4-phenoxybenzenesulfonyl)-1,2,3,4-tetrahydroisoquinoline-3-hydroxamic acid,
R-2-(4-phenoxybenzenesulfonyl)-1,2,3,4-tetrahydroisoquinoline-3-carboxylic acid,
R-2-(4-(4-dimethylaminophenoxy)benzenesulfonyl)-1,2,3,4-tetrahydroisoquinoline-3-hydroxamic acid,
R-2-(4-dimethylaminobiphenylsulfonyl)-1,2,3,4-tetrahydroisoquinoline-3-carboxylic acid,
R-2-(4-benzoylphenylsulfonyl)-1,2,3,4-tetrahydroisoquinoline-3-hydroxamic acid,
R-2-(4-methoxybenzenesulfonyl)-7-hydroxy-1,2,3,4-tetrahydroisoquinoline-3-hydroxamic acid,
R-2-(4-methoxybenzenesulfonyl)-7-nitro-1,2,3,4-tetrahydroisoquinoline-3-hydroxamic acid,
2-(4-methoxybenzenesulfonyl)-6,7-propylene-1,2,3,4-tetrahydroisoquinoline-1-hydroxamic acid,
R-5-(4-methoxybenzenesulfonyl)-4,5,6,7-tetrahydro-1H-imidazo-(4,5-c)-pyridine-6-hydroxamic acid,
R-2-(4-methoxybenzenesulfonyl)-1,2,3,4-tetrahydro-9H-pyrido-(3,4-c)-indole-3-hydroxamic acid,
R-2-(4-phenoxybenzenesulfonyl)-1,2,3,4-tetrahydro-9H-pyrido-(3,4-c)-indole-3-hydroxamic acid are used.

4. The compound of the formula I as claimed in one or more of claims 1 to 3, wherein the central carbon atom between amino and hydroxamic acid group is present as R enantiomer.

5. A process for preparing the compound of the formula I as claimed in one or more of claims 1 to 4, which comprises
a) reacting an imino acid of the formula XI 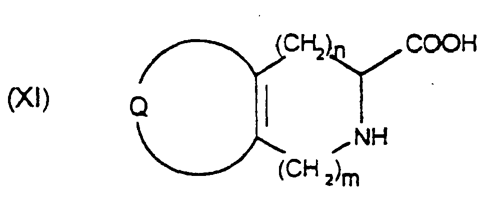 where the radical Q and n and m are defined as the formula I with a (C₁-C₄)-alcohol or a benzyl alcohol to give the compound of the formula XII 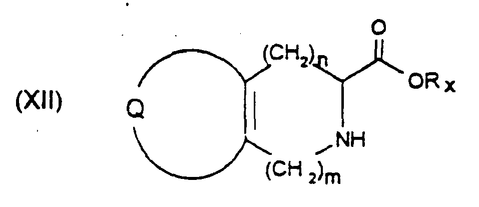 where Rₓ is (C₁-C₄)-alkyl or benzyl, or
b) reacting a compound of the formula XII prepared according to process a) with the compound of the formula XIII 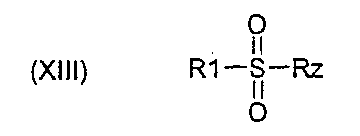 where R¹ is as defined in formula I and R_{z} is a chlorine atom, imidazolyl or -OH, in the presence of a base to give a compound of the formula XIV 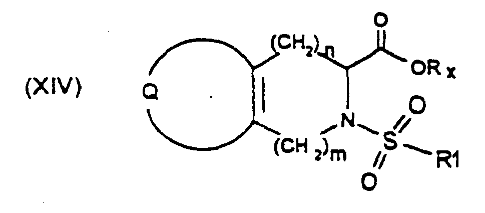 where Q, R1, n and m are defined as the formula I and Rx is as defined in formula XII, or
c) reacting a compound of the formula XII prepared according to process a) with a base and subsequently with a compound of the formula XIII to give a compound of the formula XIV, or
d) reacting a compound of the formula XI with a compound of the formula XIII to give a compound of the formula XV 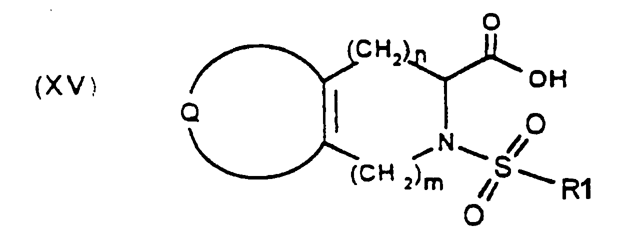 where Q, R¹, n and m are as defined in formula I, or
e) reacting a compound of the formula XIV to give a compound XV, or
f) reacting a compound of the formula XIV prepared according to process b) or c) with the hydroxylamine of the formula XVI
(XVI) H₂N-ORy
where Ry is a hydrogen atom or an oxygen group, to give the compound of the formula I and, if appropriate, removing the protective group for oxygen, or
g) reacting a compound of the formula XV prepared according to process d) or e) with the hydroxylamine of the formula XVI to give the compound of the formula I, or
h) separating into the pure enantiomers a compound of the formula I prepared according to process f) or g) which, owing to its chemical structure, exists in enantiomeric forms, by forming salts with enantiomerically pure acids or bases, by chromatography using chiral stationary phases or derivatization by means of chiral enantiomerically pure compounds such as amino acids, separation of the resulting diastereomers, and removal of the chiral auxiliary, or
i) isolating the compound of the formula I prepared according to processes f), g) or h) either in free form or, if acidic or basic groups are present, converting it, if appropriate, into physiologically tolerable salts.

6. A pharmaceutical, comprising an efficacious amount of at least one compound of the formula I 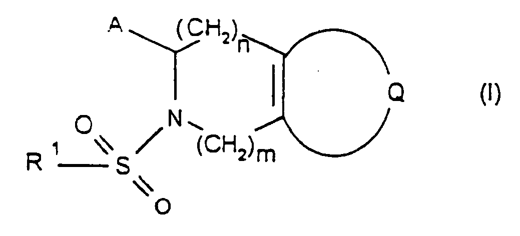 and/or an optionally stereoisomeric form of the compound of the formula I and/or a physiologically tolerable salt of the compound of the formula I,
where in the case i)
R¹ is
a) a radical of the formula II 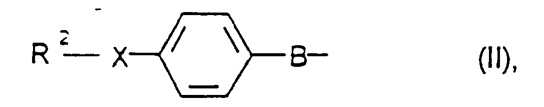
b) a radical of the formula III 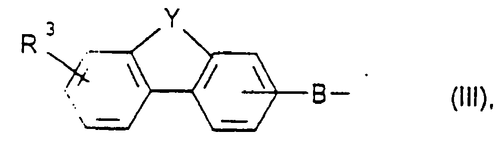
c) a radical of the formula IV 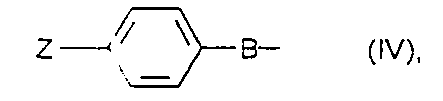 where Z is a radical of a heterocycle or a substituted heterocycle such as
1) pyrrole,
2) thiazole,
3) pyrazole,
4) pyridine,
5) imidazole,
6) pyrrolidine,
7) piperidine,
8) thiophene,
9) oxazole,
10) isoxazole,
11) morpholine or
12) piperazine, or
d) a radical of the formula V 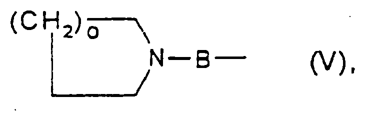 where o is the number 1 or 2 and one of the carbon atoms in the ring may be replaced by -O- or -S-, and
Q as part of the structural formula I 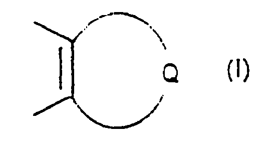
1) is the structural moiety VI 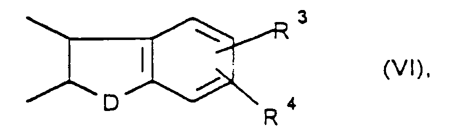
2) the structural moiety VII 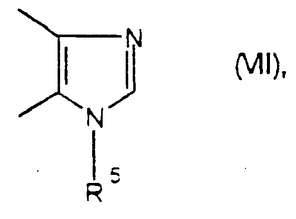
3) is the structural moiety VIII 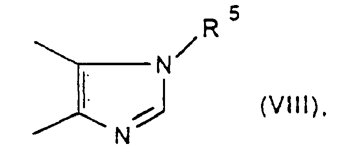
4) the structural moiety IX 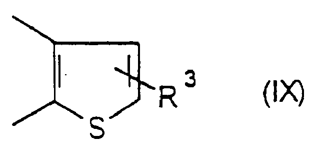 or
5) is the structural moiety X 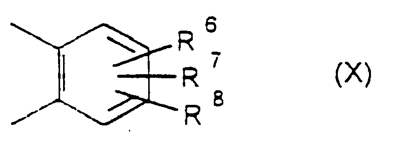 where D is NR⁴ or S,
R² is
1) phenyl or
2) phenyl which is mono- to trisubstituted by
2.1 hydroxyl,
2.2 -O-R¹⁰, where R¹⁰
1) is (C₁-C₆)-alkyl,
2) is (C₃-C₆)-cycloalkyl,
3) is benzyl or
4) is phenyl,
2.3 -COOH,
2.4 (C₁-C₆)-alkyl,
2.5 (C₃-C₆)-cycloalkyl-O-(C₁-C₄)-alkyl,
2.6 halogen,
2.7 -CN,
2.8 -NO₂,
2.9 -CF₃,
2.10 -O-C(O)-R¹⁰ and R¹⁰ is as defined above,
2.11 -O-C(O)-phenyl, mono- or disubstituted by R³,
2.12 -C(O)-O-R¹⁰ and R¹⁰ is as defined above,
2.13 methylenedioxo,
2.14 -C (O) -NR¹¹R¹², where
R¹¹ and R¹² may be identical or different and each is
1) a hydrogen atom,
2) (C₁-C₄) -alkyl or
3) benzyl or
4) R¹¹ and R¹² together with the linking nitrogen atom form a pyrrolidine, piperidine, morpholine or piperazine radical, or
2.15 -NR¹³R¹⁴, where R¹³ is a hydrogen atom or (C₁-C₄)-alkyl and
R¹⁴
1) is a hydrogen atom,
2) is (C₁-C₄) -alkyl,
3) is benzyl,
4) is -C(O)-R¹⁰ or
5) is -C(O)-O-R¹⁰ and R¹⁰ is as defined above,
R³ and R⁴ are identical or different and each is
1) a hydrogen atom,
2) (C₁-C₅)-alkyl,
3) (C₁-C₅)-alkoxy,
4) halogen,
5) hydroxyl,
6) -O-C(O)-R¹⁰ and R¹⁰ is as defined above, or
7) R³ and R⁴ together form the radical -O-CH₂-O-,
R⁵ is
a) a hydrogen atom,
b) (C₁-C₅)-alkyl or
c) benzyl, and
- R⁶, R⁷ and R⁸ are identical or different and each is
a) a hydrogen atom, or
b) has, in the case of i), the meaning of R² under items 2.1 to 2.14, and
n is zero, 1 or 2,
m is zero, 1 or 2, the sum of n and m being 1, 2 or 3, or
where in the case ii)
R¹ is
1) phenyl or
2) phenyl, mono- to trisubstituted by R², where R² is as defined for the case i) under items 2.1 to 2.15,
Q is the structural moiety X and
R⁶, R⁷ and R⁸ are identical or different and each is defined as above,
n is 1 and
m is 1, or
where in the case iii)
R¹, Q, R⁶, R⁷ and R⁸ are identical or different and each has the meaning mentioned for the case ii), m and n are zero, 1 or 2 and where the meanings of n and m are not identical, and
X is
a) a covalent bond,
b) -O-,
c) -S-,
d) -S(O)-,
e) -S(O)₂-,
f) -C(O)- or
g) -C(OH)-, and
Y is
a) -O- or
b) -S-, and
A is HO-NH-C(O)- or HO-C(O)- and
B is a) -(CH₂)_{q}-, where q is zero, 1, 2, 3 or 4, or b) is -CH=CH-, together with physiologically acceptable auxiliaries and excipients, optionally further additives and/or other active compounds.

7. The pharmaceutical as claimed in claim 6, comprising an effective amount of at least one compound of the formula I, where
R¹ in the case i) is a radical of the formula II or III and Q is the structural moiety VI, VII, VIII or X,
R¹ in the case ii) is phenyl or phenyl, mono- to trisubstituted by methoxy, and Q is the structural moiety X, or
R¹ in the case iii) is phenyl, Q is the structural moiety X, n is zero and m is 2, and
A is HO-NH-C(O)- or HO-C(O)-,
B is a covalent bond,
X is an oxygen atom or a covalent bond, and
R² is phenyl or phenyl substituted by
a) hydroxyl,
b) -O-R¹⁰, where R¹⁰ is (C₁-C₃)-alkyl or benzyl,
c) (C₁-C₂)-alkyl,
d) fluorine or chlorine,
e) -CN,
f) -CF₃ or
g) -NR¹³R¹⁴, where R¹³ and R¹⁴ are each (C₁-C₃) - alkyl,
R³, R⁴, R⁵, R⁶, R⁷ and R⁸ are identical or different and each is
a) a hydrogen atom,
b) methoxy,
c) methylenedioxo,
d) amino or
e) hydroxyl.

8. The pharmaceutical as claimed in claim 6 or 7, comprising an effective amount of at least one compound of the formula I where
R-2-(biphenylsulfonyl)-1,2,3,4-tetrahydroisoquinoline-3-hydroxamic acid,
R-2-(4-chlorobiphenylsulfonyl)-1,2,3,4-tetrahydroisoquinoline-3-hydroxamic acid,
R-2-(4-chlorobiphenylsulfonyl)-1,2,3,4-tetrahydroisoquinoline-3-carboxylic acid,
R-2-(4-phenoxybenzenesulfonyl)-1,2,3,4-tetrahydroisoquinoline-3-hydroxamic acid,
R-2-(4-phenoxybenzenesulfonyl)-1,2,3,4-tetrahydroisoquinoline-3-carboxylic acid,
R-2-(4-(4-dimethylaminophenoxy)benzenesulfonyl)-1,2,3,4-tetrahydroisoquinoline-3-hydroxamic acid,
R-2-(4-dimethylaminobiphenylsulfonyl)-1,2,3,4-tetrahydroisoquinoline-3-carboxylic acid,
R-2-(4-benzoylphenylsulfonyl)-1,2,3,4-tetrahydroisoquinoline-3-hydroxamic acid,
R-2-(4-methoxybenzenesulfonyl)-7-hydroxy-1,2,3,4-tetrahydroisoquinoline-3-hydroxamic acid,
R-2-(4-methoxybenzenesulfonyl)-7-nitro-1,2,3,4-tetrahydroisoquinoline-3-hydroxamic acid,
2-(4-methoxybenzenesulfonyl)-6,7-propylene-1,2,3,4-tetrahydroisoquinoline-1-hydroxamic acid,
R-5-(4-methoxybenzenesulfonyl)-4,5,6,7-tetrahydro-1H-imidazo-(4,5-c)-pyridine-6-hydroxamic acid,
R-2-(4-methoxybenzenesulfonyl)-1,2,3,4-tetrahydro-9H-pyrido-(3,4-c)-indole-3-hydroxamic acid or
R-2-(4-phenoxybenzenesulfonyl)-1,2,3,4-tetrahydro-9H-pyrido-(3,4-c)-indole-3-hydroxamic acid are used.

9. The pharmaceutical as claimed in one or more of claims 6 to 8, comprising an effective amount of at least one compound of the formula I, where the central carbon atom between amino and hydroxamic acid group is present as R enantiomer.

10. The use of at least one compound of the formula I as claimed in one or more of claims 6 to 9 for preparing pharmaceuticals for the prophylaxis and therapy of disorders in the course of which an increased activity of matrix-degrading metalloproteinases is involved.

11. The use as claimed in claim 10 for the treatment of disorders of the connective tissue such as collagenoses, periodontal disorders, wound healing disorders or chronic disorders of the locomotory apparatus such as inflammatorily, immunologically or metabolically related acute and chronic arthritides, arthropathies, myalgias and disorders of the bone metabolism or degenerative joint disorders such as osteoarthroses, spondyloses, chondrolysis after joint trauma or relatively long joint immobilization after meniscus or patella injuries or ligament tears, or for the treatment of ulceration, artherosclerosis and stenoses, or inhibition of the release of tumor necrosis factor, or for the treatment of inflammations, carcinomataceous disorders, formation of tumor metastases, cachexia, anorexia and septic shock.

12. A process for the production of a pharmaceutical as claimed in claims 6 to 9, which comprises bringing into a suitable administration form at least one compound of the formula I as claimed in one or more of claims 6 to 9 and/or at least one physiologically tolerable salt of the compound of the formula I and/or an optionally stereoisomeric form of the compound of the formula I, using physiologically acceptable auxiliaries and excipients and, if appropriate, further additives and/or other active compounds.

## Revendications

1. Composé de formule I 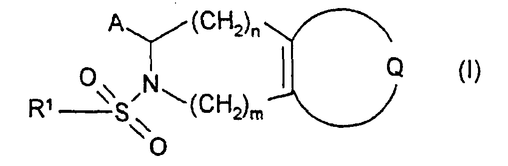 et/ou forme éventuellement stéréo-isomère du composé de formule I et/ou sel physiologiquement acceptable du composé de formule I, dans le cas i)
R¹ représentant
a) un radical de formule II 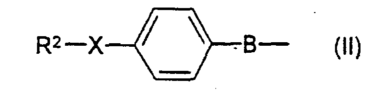
b) un radical de formule III 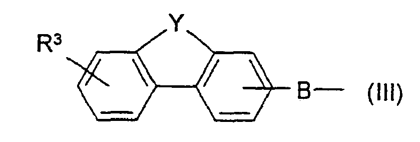
c) un radical de formule IV 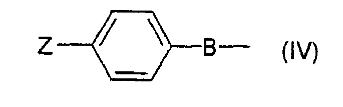 où Z est un hétérocycle ou hétérocycle substitué, tel que
1) pyrrole,
2) thiazole,
3) pyrazole,
4) pyridine,
5) imidazole,
6) pyrrolidine,
7) pipéridine,
8) thiophène,
9) oxazole,
10) isoxazole,
11) morpholine ou
12) pipérazine, ou
d) un radical de formule V 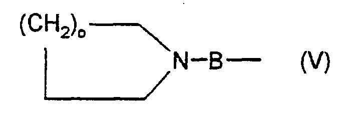 dans laquelle o est le nombre 1 ou 2, et l'un des atomes de carbone dans le cycle est éventuellement remplacé par -O- ou -S-, et
Q en tant que partie de la formule développée I 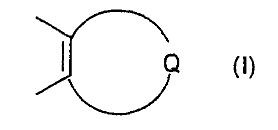 représentant
1) le fragment VI 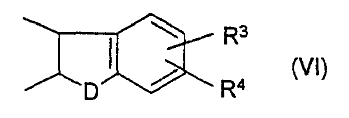
2) le fragment VII 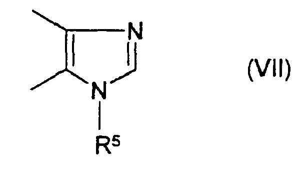
3) le fragment VIII 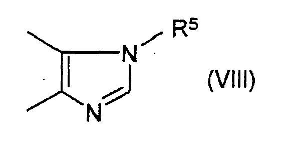
4) le fragment IX 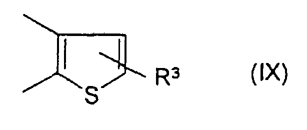 ou
5) le fragment X 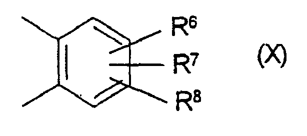 où D représente NR⁴ ou S,
R² représente
1) le groupe phényle ou
2) un groupe phényle une à trois fois substitué par
2.1 hydroxy,
2.2. -O-R¹⁰, où R¹⁰ représente
1) un groupe alkyle en C₁-C₆,
2) un groupe cycloalkyle en C₃-C₆,
3) le groupe benzyle ou
4) le groupe phényle,
2.3. -COOH,
2.4 alkyle en C₁-C₆,
2.5 cycloalkyl (C₃-C₆)-O-alkyle (C₁-C₄)
2.6 halogène
2.7 -CN,
2.8 -NO₂,
2.9 -CF₃,
2.10. -O-C (O) -R¹⁰ et R¹⁰ est tel que défini plus haut,
2.11. -O-C(O)-phényle, une ou deux fois substitué par R³,
2.12 . -C(O)-O-R¹⁰ et R¹⁰ est tel que défini plus haut,
2.13. méthylènedioxo,
2.14. -C(O)-NR¹¹R¹², où
R¹¹ et R¹² peuvent être identiques ou différents et représentent
1) un atome d'hydrogène,
2) un groupe alkyle en C₁-C₄ ou
3) le groupe benzyle ou
4) R¹¹ et R¹² forment ensemble, avec l'atome d'azote auquel il sont fixés, un radical pyrrolidino, pipéridino, morpholino ou pipérazino,
2.15. NR¹³R¹⁴, où R¹³ représente
un atome d'hydrogène ou un groupe alkyle en C₁-C₄ et
R¹⁴ représente
1) un atome d'hydrogène,
2) un groupe alkyle en C₁-C₄,
3) le groupe benzyle,
4) -C(O)-R¹⁰ ou
5) -C(O)-O-R¹⁰ et R¹⁰ est tel que défini plus haut,
R³ et R⁴ sont identiques ou différents, et représentent
1) un atome d'hydrogène,
2) un groupe alkyle en C₁-C₅,
3) un groupe alcoxy en C₁-C₅,
4) un atome d'halogène,
5) un groupe hydroxy,
6) -O-C(O)-R¹⁰ et R¹⁰ est tel que défini plus haut, ou
7) R³ et R⁴ forment ensemble le radical -O-CH₂-O-,
R⁵ représente
a) un atome d'hydrogène,
b) un groupe alkyle en C₁-C₅ ou
c) le groupe benzyle, et
R⁶, R⁷ et R⁸ sont identiques ou différents, et
a) représentent un atome d'hydrogène, ou
b) ont la signification pour le cas i) de R² aux points 2.1 à 2.14, et
n représente zéro, 1 ou 2,
m représente zéro, 1 ou 2, la somme de n et m étant 1, 2 ou 3,
à l'exclusion du cas où
A représente HO-C(O)-, Q en tant que partie de la formule développée I représente le fragment X, R⁶,
R⁷ et R⁸ représentent un atome d'hydrogène ou le groupe -O-méthyle, n représente 1, m représente 1 et R¹ représente le radical de formule V, où o est le nombre 2 et l'un des atomes de carbone est remplacé par -O-, ou
dans le cas ii)
R¹ représentant
1) le groupe phényle ou
2) un groupe phényle une à trois fois substitué par R², R² étant défini comme pour le cas i) aux points 2.1 à 2.15,
Q représentant le fragment X et
R⁶, R⁷ et R⁸ sont identiques ou différents, et sont tels que définis plus haut,
n représente 1 et
m représente 1,
à l'exclusion du cas où
A représente HO-C(O)-, R⁶, R⁷ et R⁸ représentent un atome d'hydrogène, n représente 1, m représente 1 et R¹ représente un groupe phényle qui est monosubstitué par un groupe méthyle, nitro ou amino, ou
A représente HO-C(O)-, R⁶, R⁷ et R⁸ représentent un atome d'hydrogène, n représente 1, m représente 1 et R¹ représente un groupe phényle qui est substitué deux fois par un groupe amino et un atome de chlore, ou
dans le cas iii)
R¹, Q, R⁶, R⁷ et R⁸ étant identiques ou différents, et ayant les significations indiquées pour le cas ii), m et n représentant zéro, 1 ou 2 et la signification de n et m n'étant pas la même,
à l'exclusion du cas où
A représente HO-C(O)-, R⁶, R⁷ et R⁸ représentent un atome d'hydrogène, n représente 1, m représente zéro et R¹ représente un groupe phényle qui est monosubstitué par un groupe méthyle,
et X représentant
a) une liaison covalente,
b) -O-,
c) -S-,
d) -S(O)-,
e) -S(O)₂,
f) -C(O)- ou
g) -C(OH)- et
Y représentant
a) -O-,
b) -S- et
A représentant HO-NH-C(O)- ou HO-C(O)- et
B représentant a) -(CH₂)_{q}- où q représente zéro, 1, 2, 3 ou 4, ou b) -CH=CH-.

2. Composé de formule I selon la revendication 1 et/ou sel physiologiquement acceptable du composé de formule I et/ou forme éventuellement stéréo-isomère du composé de formule I,
R¹ représentant dans le cas i) un radical de formule II ou III et Q représentant le fragment VI, VII, VIII ou X,
R¹ représentant dans le cas ii) le groupe phényle ou un groupe phényle une à trois fois substitué par méthoxy, et Q représentant le fragment X,
R¹ représentant dans le cas iii) le groupe phényle, Q représentant le fragment X, n représentant zéro et m représentant 2, et
A représentant HO-NH-C(O)- ou HO-C(O)-,
B représentant une liaison covalente,
X représentant un atome d'oxygène ou une liaison covalente, et
R² représentant le groupe phényle ou un groupe phényle par
a) hydroxy,
b) O-R¹⁰, où R¹⁰ représente un groupe alkyle en C₁-C₃ ou benzyle,
c) alkyle en C₁-C₂,
d) le fluor ou le chlore,
e) -CN,
f) -CF₃ ou
g) -NR¹³R¹⁴, où R¹³ et R¹⁴ représentent un groupe alkyle en C₁-C₃
R³, R⁴, R⁵, R⁶, R⁷ et R⁸ étant identiques ou différents, et représentant
a) un atome d'hydrogène,
b) le groupe méthoxy,
c) le groupe méthylènedioxo,
d) le groupe amino ou
e) le groupe hydroxy.

3. Composé de formule selon la revendication 1 ou 2, qui est
l'acide R-2-(biphénylsulfonyl)-1,2,3,4-tétrahydro-isoquinoléine-3-hydroxamique,
l'acide R-2-(4-chloro-biphénylsulfonyl)-1,2,3,4-tétrahydro-isoquinoléine-3-hydroxamique,
l'acide R-2-(4-chloro-biphénylsulfonyl)-1,2,3,4-tétrahydro-isoquinoléine-3-carboxylique,
l'acide R-2-(4-phénoxybenzènesulfonyl)-1,2,3,4-tétrahydro-isoquinoléine-3-hydroxamique,
l'acide R-2-(4-phénoxybenzènesulfonyl)-1,2,3,4-tétrahydro-isoquinoléine-3-carboxylique,
l'acide R-2-(4-(4-diméthylaminophénoxy)-benzènesulfonyl)-1,2,3,4-tétrahydro-isoquinoléine-3-hydroxamique,
l'acide R-2-(4-diméthylaminobiphényl-sulfonyl)-1,2,3,4-tétrahydro-isoquinoléine-3-carboxylique,
l'acide R-2-(4-benzoylphénylsulfonyl)-1,2,3,4-tétrahydro-isoquinoléine-3-hydroxamique,
l'acide R-2-(4-méthoxybenzènesulfonyl)-7-hydroxy-1,2,3,4-tétrahydro-isoquinoléine-3-hydroxamique,
l'acide R-2-(4-méthoxybenzènesulfonyl)-7-nitro-1,2,3,4-tétrahydro-isoquinoléine-3-hydroxamique,
l'acide 2-(4-méthoxybenzènesulfonyl)-6,7-propylène-1,2,3,4-tétrahydro-isoquinoléine-1-hydroxamique,
l'acide R-5-(4-méthoxybenzènesulfonyl)-4,5,6,7-tétrahydro-1H-imidazo-(4,5-c)-pyridine-6-hydroxamique,
l'acide R-2-(4-méthoxybenzènesulfonyl)-1,2,3,4-tétrahydro-9H-pyrido-(3,4-c)-indole-3-hydroxamique,
l'acide R-2-(4-phénoxybenzènesulfonyl)-1,2,3,4-tétrahydro-9H-pyrido-(3,4-c)-indole-3-hydroxamique,

4. Composés de formule I selon une ou plusieurs des revendications 1 à 3, **caractérisés en ce que** l'atome de carbone central entre les groupes amino et hydroxamique se trouve sous forme d'énantio-mère R.

5. Procédé pour la préparation du composé de formule I selon une ou plusieurs des revendications 1 à 4, **caractérisé en ce que**
a) on fait réagir un iminoacide de formule XI 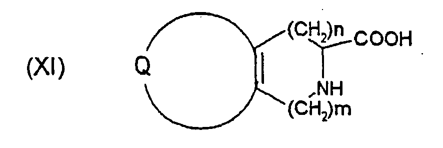 dans laquelle Q ainsi que n et m sont tels que définis dans la formule I, avec un alcool en C₁-C₄ ou un alcool benzylique, pour obtenir le composé de formule XII, 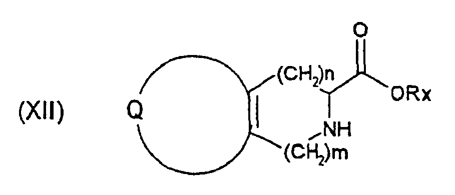 dans laquelle Rₓ représente un groupe alkyle en C₁-C₄ ou benzyle, ou
b) on fait réagir un composé de formule XII, préparé selon le procédé a), avec le composé de formule XIII, 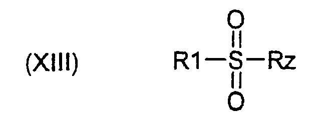 dans laquelle R¹ est défini comme dans la formule I et R_{z} représente un atome de chlore, le groupe imidazoyle ou -OH, en présence d'une base, pour obtenir un composé de formule XIV, 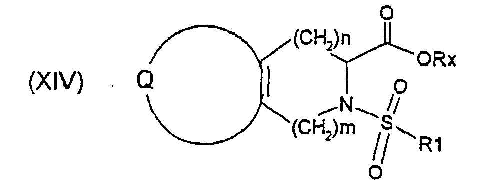 dans laquelle Q, R¹, n et m sont tels que définis dans la formule I et Rₓ est tel que défini dans la formule XII, ou
c) on fait réagir un composé de formule XII, préparé selon le procédé a), avec une base et ensuite avec un composé de formule XIII, pour obtenir un composé de formule XIV, ou
d) on fait réagir un composé de formule XI avec un composé de formule XIII pour obtenir un composé de formule XV 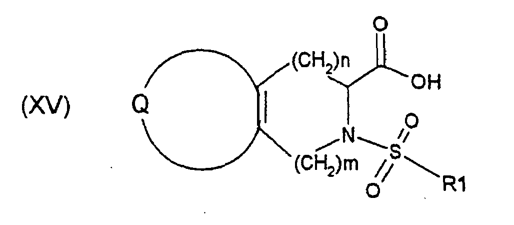 dans laquelle Q, R¹, n et m sont tels que définis dans la formule I, ou
e) on convertit un composé de formule XIV en un composé de formule XV, ou
f) on fait réagir un composé de formule XIV, préparé selon le procédé b) ou c), avec l'hydroxylamine de formule XVI,
(XVI) H₂N-OR_{y}
dans laquelle R_{y} représente un atome d'hydrogène ou un groupe protecteur d'atome d'oxygène, pour obtenir le composé de formule I et éventuellement on élimine le groupe protecteur d'atome d'oxygène, ou
g) on fait réagir un composé de formule XV, préparé selon le procédé d) ou e), avec l'hydroxylamine de formule XVI, pour obtenir le composé de formule I, ou
h) on sépare un composé de formule I, préparé selon le procédé f) ou g), qui, en raison de sa structure chimique, apparaît sous des formes énantiomères, par salification avec des acides ou des bases sous forme d'énantiomères purs, chromatographie sue des phases stationnaires chirales ou transformation en dérivés au moyen de composés sous forme d'énantiomères purs, tels que des aminoacides, séparation des diastéréoisomères ainsi obtenus et élimination du groupe auxiliaire chiral, en les énantiomères purs, ou
i) le composé de formule I, préparé selon les procédés f), g) et h) est soit isolé sous forme libre, soit, dans le cas de la présence de groupes acides ou basiques, éventuellement converti en sels physiologiquement acceptables.

6. Médicament contenant une quantité efficace d'au moins un composé de formule I 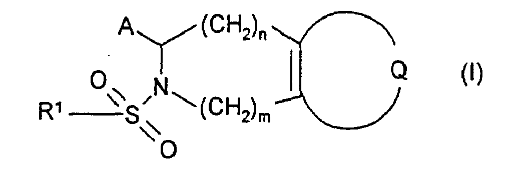 et/ou d'une forme éventuellement stéréo-isomère du composé de formule I et/ou d'un sel physiologiquement acceptable du composé de formule I, dans le cas i)
R¹ représentant
a) un radical de formule II 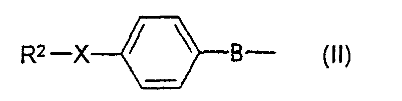
b) un radical de formule III 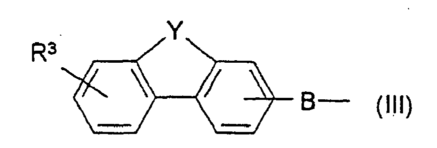
c) un radical de formule IV 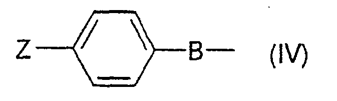 où Z est un hétérocycle ou hétérocycle substitué, tel que
1) pyrrole,
2) thiazole,
3) pyrazole,
4) pyridine,
5) imidazole,
6) pyrrolidine,
7) pipéridine,
8) thiophène,
9) oxazole,
10) isoxazole,
11) morpholine ou
12) pipérazine, ou
d) un radical de formule V 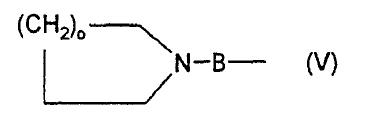 dans laquelle o est le nombre 1 ou 2, et l'un des atomes de carbone dans le cycle est éventuellement remplacé par -O- ou -S-,
Q en tant que partie de la formule développée I 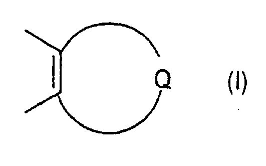 représentant
1) le fragment VI 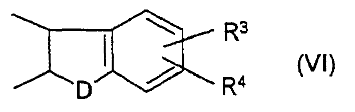
2) le fragment VII 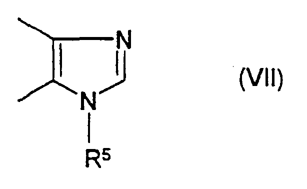
3) le fragment VIII 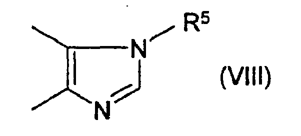
4) le fragment IX 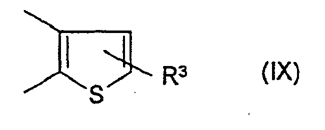 ou
5) le fragment X 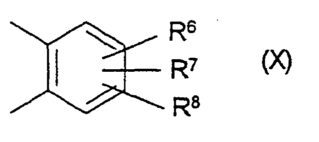 où D représente NR⁴ ou S,
R² représente
1) le groupe phényle ou
2) un groupe phényle une à trois fois substitué par
2.1 hydroxy,
2.2. -O-R¹⁰, où R¹⁰représente
1) un groupe alkyle en C₁-C₆,
2) un groupe cycloalkyle en C₃-C₆,
3) le groupe benzyle ou
4) le groupe phényle,
2.3. -COOH,
2.4 alkyle en C₁-C₆,
2.5 cycloalkyl(C₃-C₆)-O-alkyle(C₁-C₄),
2.6 halogène
2.7 -CN,
2.8 -NO₂,
2.9 -CF₃,
2.10. -O-C(O)-R¹⁰ et R¹⁰ est tel que défini plus haut,
2.11. -O-C(O)-phényle, une ou deux fois substitué par R³,
2.12. -C(O)-O-R¹⁰ et R¹⁰ est tel que défini plus haut,
2.13. méthylènedioxo,
2.14. -C(O)-NR¹¹R¹², où
R¹¹ et R¹² peuvent être identiques ou différents et représentent
1) un atome d'hydrogène,
2) un groupe alkyle en C₁-C₄ ou
3) le groupe benzyle ou
4) R¹¹ et R¹² forment ensemble, avec l'atome d'azote auquel il sont fixés, un radical pyrrolidino, pipéridino, morpholino ou pipérazino,
2.15. NR¹³R¹⁴, où R¹³ représente
un atome d'hydrogène ou un groupe alkyle en C₁-C₄ et
R¹⁴ représente
1) un atome d'hydrogène,
2) un groupe alkyle en C₁-C₄,
3) le groupe benzyle,
4) -C(O)-R¹⁰ ou
5) -C(O)-O-R¹⁰ et R¹⁰ est tel que défini plus haut,
R³ et R⁴ sont identiques ou différents, et représentent
1) un atome d'hydrogène,
2) un groupe alkyle en C₁-C₅,
3) un groupe alcoxy en C₁-C₅,
4) un atome d'halogène,
5) un groupe hydroxy,
6) -O-C(O)-R¹⁰ et R¹⁰ est tel que défini plus haut, ou
7) R³ et R⁴ forment ensemble le radical -O-CH₂-O-,
R⁵ représente
a) un atome d'hydrogène,
b) un groupe alkyle en C₁-C₅,
c) le groupe benzyle, et
R⁶, R⁷ et R⁸ sont identiques ou différents, et
a) représentent un atome d'hydrogène, ou
b) ont la signification pour le cas i) de R² aux points 2.1 à 2.14, et
n représente zéro, 1 ou 2,
m représente zéro, 1 ou 2, la somme de n et m étant 1, 2 ou 3,
ou
dans le cas ii)
R¹ représentant
1) le groupe phényle ou
2) un groupe phényle une à trois fois substitué par R², R² étant défini comme pour le cas i) aux points 2.1 à 2.15,
Q représentant le fragment X et
R⁶, R⁷ et R⁸ sont identiques ou différents, et sont tels que définis plus haut,
n représente 1 et
m représente 1,
ou
dans le cas iii)
R¹, Q, R⁶, R⁷ et R⁸ étant identiques ou différents, et ayant les significations indiquées pour le cas ii), m et n représentant zéro, 1 ou 2 et la signification de n et m n'étant pas la même,
X représentant
a) une liaison covalente,
b) -O-,
c) -S-,
d) -S(O)-,
e) -S(O)₂-,
f) -C(O)- ou
g) -C(OH)- et
Y représentant
a) -O-,
b) -S- et
A représentant HO-NH-C(O)- ou HO-C(O)- et
B représentant a) -(CH₂)_{q}- où q représente zéro, 1, 2, 3 ou 4, ou b) -CH=CH-,
en plus d'adjuvants et de véhicules physiologiquement acceptables, éventuellement d'autres additifs et/ou d'autres substances actives.

7. Médicament selon la revendication 6, contenant une quantité efficace d'au moins un composé de formule I
R¹ représentant dans le cas i) un radical de formule II ou III et Q représentant le fragment VI, VII, VIII ou X,
R¹ représentant dans le cas ii) le groupe phényle ou un groupe phényle une à trois fois substitué par méthoxy, et Q représentant le fragment X,
R¹ représentant dans le cas iii) le groupe phényle, Q représentant le fragment X, n représentant zéro et m représentant 2, et
A représentant HO-NH-C(O)- ou HO-C(O)-,
B représentant une liaison covalente,
X représentant un atome d'oxygène ou une liaison covalente, et
R² représentant le groupe phényle ou un groupe phényle par
a) hydroxy,
b) O-R¹⁰, où R¹⁰ représente un groupe alkyle en C₁-C₃ ou benzyle,
c) alkyle en C₁-C₂,
d) le fluor ou le chlore,
e) -CN,
f) -CF₃ ou
g) -NR¹³R¹⁴, où R¹³ et R¹⁴ représentent un groupe alkyle en C₁-C₃
R³ R⁴ R⁵ R⁶ R⁷ et R⁸ étant identiques ou différents, et représentant
a) un atome d'hydrogène,
b) le groupe méthoxy,
c) le groupe méthylènedioxo,
d) le groupe amino ou
e) le groupe hydroxy.

8. Médicament selon la revendication 6 ou 7, contenant une quantité efficace d'au moins un composé de formule I, dans lequel on utilise
l'acide R-2-(biphénylsulfonyl)-1,2,3,4-tétrahydro-isoquinoléine-3-hydroxamique,
l'acide R-2-(4-chloro-biphénylsulfonyl)-1,2,3,4-tétrahydro-isoquinoléine-3-hydroxamique,
l'acide R-2-(4-chloro-biphénylsulfonyl)-1,2,3,4-tétrahydro-isoquinoléine-3-carboxylique,
l'acide R-2-(4-phénoxybenzènesulfonyl)-1,2,3,4-tétrahydro-isoquinoléine-3-hydroxamique,
l'acide R-2-(4-phénoxybenzènesulfonyl)-1,2,3,4-tétrahydro-isoquinoléine-3-carboxylique,
l'acide R-2-(4-(4-diméthylaminophénoxy)-benzènesulfonyl)-1,2,3,4-tétrahydro-isoquinoléine-3-hydroxamique,
l'acide R-2-(4-diméthylaminobiphényl-sulfonyl)-1,2,3,4-tétrahydro-isoquinoléine-3-carboxylique,
l'acide R-2-(4-benzoylphénylsulfonyl)-1,2,3,4-tétrahydro-isoquinoléine-3-hydroxamique,
l'acide R-2-(4-méthoxybenzènesulfonyl)-7-hydroxy-1,2,3,4-tétrahydro-isoquinoléine-3-hydroxamique,
l'acide R-2-(4-méthoxybenzènesulfonyl)-7-nitro-1,2,3,4-tétrahydro-isoquinoléine-3-hydroxamique,
l'acide 2-(4-méthoxybenzènesulfonyl)-6,7-propylène-1,2,3,4-tétrahydro-isoquinoléine-1-hydroxamique,
l'acide R-5-(4-méthoxybenzènesulfonyl)-4,5,6,7-tétrahydro-1H-imidazo-(4,5-c)-pyridine-6-hydroxamique,
l'acide R-2-(4-méthoxybenzènesulfonyl)-1,2,3,4-tétrahydro-9H-pyrido-(3,4-c)-indole-3-hydroxamique,
l'acide R-2-(4-phénoxybenzènesulfonyl)-1,2,3,4-tétrahydro-9H-pyrido-(3,4-c)-indole-3-hydroxamique,

9. Médicament selon une ou plusieurs des revendications 6 à 8, contenant une quantité efficace d'au moins un composé de formule I, dans lequel l'atome de carbone central entre les groupes amino et hydroxamique se trouve sous forme d'énantiomère R.

10. Utilisation d'au moins un composé de formule I selon une ou plusieurs des revendications 6 à 9, pour la fabrication de médicaments destinés à la prophylaxie et la thérapie de maladies au cours desquelles est impliquée une activité accentuée de métalloprotéases dégradant la matrice.

11. Utilisation selon la revendication 10, pour le traitement de maladies du tissu conjonctif, telles que les collagénoses, les maladies périodontaires, les troubles de la cicatrisation ou des maladies chroniques de l'appareil moteur, telles que des arthrites, arthropathies, myalgies aiguës et chroniques, inflammatoires, immunologiques ou d'origine métabolique et des troubles du métabolisme osseux ou des affections articulaires dégénératives telles que les ostéoarthroses, les spondyloses, la chondroporose après traumatisme articulaire ou relativement longue immobilisation d'articulations après blessures du ménisque ou de la rotule ou déchirures des ligaments ou le traitement de l'ulcération, de l'athérosclérose et de sténoses ou l'inhibition de la libération du facteur de nécrose tumorale ou le traitement d'inflammations, de maladies cancéreuses, de la formation de métastases tumorales, la cachexie, l'anorexie et le choc septique.

12. Procédé pour la fabrication d'un médicament selon les revendications 6 à 9, **caractérisé en ce qu'**on met sous une forme d'administration appropriée au moins un composé de formule I selon une ou plusieurs des revendications 6 à 9 et/ou au moins un sel physiologiquement acceptable du composé de formule I et/ou une forme éventuellement stéréoisomère du composé de formule I avec des adjuvants et véhicules physiologiquement acceptables et éventuellement d'autres additifs et/ou d'autres substances actives.
